# EUROPÄISCHE PATENTSCHRIFT

(11) **EP 0 519 463 B1**
(45) Veröffentlichungstag und Bekanntmachung des Hinweises auf die Patenterteilung: **21.10.1998**
(21) Anmeldenummer: 92110298.4
(22) Anmeldetag: 17.06.1992
(51) Int. Cl.: C07H 21/00, C12Q 1/68

(54) **Synthetische katalytische Oligonukleotide**
Synthetic, catalytic oligonucleotides
Oligonucléotides catalytiques, synthétiques

(30) Priorität: 20.06.1991 DE 4120406; 15.05.1992 DE 4216134
(43) Veröffentlichungstag der Anmeldung: 23.12.1992
(60) Teilanmeldung: 97121976.1
(73) Patentinhaber: EUROPÄISCHES LABORATORIUM FÜR MOLEKULARBIOLOGIE (EMBL), D-69117 Heidelberg (DE)
(72) Erfinder: Sproat, Brian, Dr., D-37139 Adelebsen (DE); Lamond, Angus, Dr., GB Fife DD6 8NN (Scottland) (DE); Paolella, Giovanni, Dr., W-6901 Gaiberg (DE)
(74) Vertreter: Huber, Bernhard, Dipl.-Chem.

(56) Entgegenhaltungen:
- EP-A- 0 360 257
- EP-A- 0 427 073
- EP-A- 0 427 074
- US-A- 4 469 863
- PROCEEDINGS OF THE NATIONAL ACADEMY OF SCIENCES OF USA. Bd. 87, Nr. 19, 1990, WASHINGTON US Seiten 7747 - 51 A.M.IRIBARREN ET AL. '2'-O-Alkyl Oligoribonucleotides as Antisense Probes.'
- BIOCHEMISTRY. Bd. 28, Nr. 12, 1989, EASTON, PA US Seiten 4929 - 33 A.HAMPEL ET AL. 'RNA Catalytic Properties of the Minimum (-)s TRSV Sequence.'
- NUCLEIC ACIDS RESEARCH. Bd. 19, Nr. 4, 1991, ARLINGTON, VIRGINIA US Seiten 733 - 8 B.S.SPROAT ET AL. 'New Synthetic Routes to Synthons Suitable for 2'-O-allyloligoribonucleotide Assembly.'
- NUCLEIC ACIDS RESEARCH. Bd. 18, Nr. 1, 1990, ARLINGTON, VIRGINIA US Seiten 41 - 9 B.S.SPROAT ET AL. 'New Synthetic Routes to Protected Purine 2'-O-Methylriboside-3'-O-Phosphoramidites Using a Novel Alkylation Procedure.'
- NUCLEIC ACIDS RESEARCH. Bd. 18, Nr. 2, 1990, ARLINGTON, VIRGINIA US Seiten 299 - 304 A.HAMPEL ET AL. ''Hairpin' Catalytic RNA Model: Evidence for Helices and Sequence Requirement for Substrate RNA.'
- NUCLEIC ACIDS RESEARCH. Bd. 14, Nr. 9, 1986, ARLINGTON, VIRGINIA US Seiten 3627 - 3640 C.J.HUTCHINS ET AL. 'Self-Cleavage of Plus and Minus RNA Transcripts of Avocado Sunblotch Viroid.'

## Beschreibung

Die vorliegende Erfindung betrifft synthetische katalytische Oligonukleotidstrukturen, die zur Spaltung einer Nukleinsäure-Zielsequenz geeignet sind und modifizierte Nukleotide enthalten. Die Erfindung betrifft weiterhin ein Verfahren zur Spaltung einer Nukleinsäure-Zielsequenz unter Verwendung von synthetischen katalytischen modifizierten Oligonukleotidstrukturen.

Nach Entdeckung der von RNA vermittelten Katalyse wurden verschiedene Versuche der Inaktivierung von spezifischen RNA-Molekülen in vitro und in vivo unternommen. Von besonderem Interesse hat sich die Entdeckung erwiesen, daß Hammerkopf-Ribozyme zur Entwicklung eines Vielzweckenzyms dienen können, welches zur Erkennung und Spaltung einer gegebenen spezifischen RNA zumindestens in vitro in der Lage ist (Haseloff und Gerlach (1988)). Viele interessante Einsatzmöglichkeiten für derartige Ribozyme beruhen auf ihrer Fähigkeit, eine spezifische RNA innerhalb eines gegebenen Gemisches effizient zu erkennen und zu spalten.

Hampel et al. beschreiben in der EP-A-0 360 257 einen synthetischen RNA-Katalysator zum Schneiden von RNA-Molekülen, der aus einem Substratbindeanteil und einem "Hairpin"-Anteil besteht, sowie ein für diesen RNA-Katalysator kodierendes DNA-Molekül und ein dieses DNA-Molekül enthaltenden Vektor.

Die Einsatzmöglichkeiten für RNA-Enzyme reichen von der Entwicklung von RNA-Restriktionsenzymen bis zu spezifischen Inaktivierung von Genen in der Zelle. Ein besonderes biomedizinisches Interesse beruht auf der Tatsache, daß viele Krankheiten einschließlich vieler Tumorformen in Korrelation zur Expression spezifischer Gene stehen. Die Inaktivierung solcher Gene durch Spaltung der zugehörigen mRNA würde einen möglichen Weg zur Kontrolle und schließlichen Heilung solcher Krankheiten darstellen. Ferner besteht ein großes Bedürfnis zur Entwicklung von antiviral wirksamen Arzneimitteln, wobei die RNA-Enzyme möglicherweise ein solches Mittel sein könnten, da die virale Expression selektiv durch Spaltung der viralen RNA-Moleküle blockiert werden kann. Bisherige Versuche zur Expression von Ribozymen in der Zelle durch Transfektion der Zelle mit dem entsprechenden Gen hat sich als nicht sehr wirksam erwiesen, da zur Inaktivierung der spezifischen RNA eine sehr hohe Expression erforderlich war. Wahrscheinlich ist auch die direkte Verabreichung von RNA-Molekülen aufgrund der Sensitivität von RNA gegenüber Abbau durch RNAsen und ihren Wechselwirkungen mit Proteinen unmöglich.

Es bestand daher ein sehr großes Bedürfnis, RNA-Enzyme zu entwickeln, bei denen die Nachteile des Standes der Technik mindestens teilweise beseitigt sind.

Die der Erfindung zugrundeliegende Aufgabe wird dadurch gelöst, daß man anstelle eines RNA-Moleküls einen neuen Nukleinsäuretyp verwendet, der auf artifiziellen Nukleotide mit einem 2'-Alkoxy-Substituenten beruht. Solche Moleküle sind erheblich stabiler als native RNA-Moleküle, da sie weder durch RNAsen noch durch DNAsen gespalten werden und auch weniger Wechselwirkungen mit RNA- oder DNA-bindenden Proteinen eingehen (Iribarren et al., Proc. Natl. Acad. Sci. USA 87 (1990), 7747-7751). Derartige Moleküle versprechen eine grössere Wirksamkeit in der zellulären Umgebung als entsprechende native RNA-Moleküle. Diese modifizierten Nukleinsäuren sind jedoch normalerweise nicht als Katalysatoren wirksam, es wurde jedoch überraschenderweise festgestellt, daß ihre Aktivität erhalten bleibt, wenn man eine sehr geringe Anzahl von Hydroxylresten an spezifischen Positionen des Moleküls einbaut. Dabei bleiben überraschenderweise auch ihre charakteristischen Eigenschaften, insbesondere die Stabilität und die verringerte Wechselwirkung mit Proteinen erhalten.

Ein Gegenstand der vorliegenden Erfindung ist daher eine synthetische katalytische Oligonukleotidstruktur, die zur Hybridisierung an eine und zur Spaltung einer Nukleinsäure-Zielsequenz geeignet ist und eine Oligonukleotidstruktur mit der allgemeinen Strukturformel (II) hat: 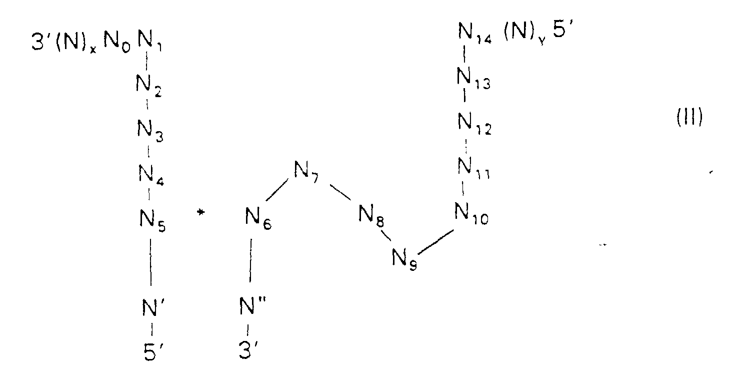 worin
N jeweils ein Nukleotid gemäß der allgemeinen Strukturformel (I) darstellt,
x und y gleich oder verschieden sein können und x ≥ 1 und y ≥ 2 ist, N₅ und N₆ zueinander jeweils komplementäre Nukleotide sind und * eine Basenpaarung darstellt,
N' und N" entweder zwei Nukleotidsequenzen darstellen, die mindestens teilweise zueinander komplementäre Nukleotide enthalten, so daß eine stabile Basenpaarung zwischen den beiden Nukleotidsequenzen ermöglicht wird,
oder
N' und N" zusammen eine einzige Nukleotidsequenz darstellen, wobei mindestens ein Teil der Sequenz durch Basenpaarung zwischen komplementären Nukleotiden einen doppelsträngigen Stamm bilden kann,
und worin gegebenenfalls ein oder mehrere zusätzliche Nukleotide N nach N₇ oder/und N₉ insertiert werden können,
und worin die Oligonukleotidstruktur Nukleotide mit der allgemeinen Strukturformel (I) enthält: 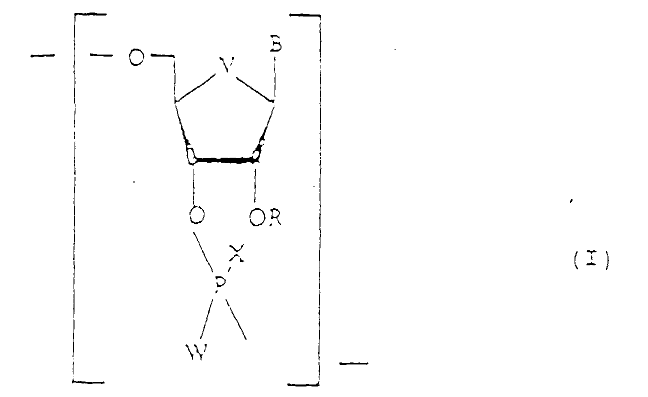
worin
B eine Nukleosidbase darstellt, die insbesondere aus der Gruppe bestehend aus Adenin-9-yl (A); Cytosin-1-yl (C), Guanin-9-yl (G), Uracil-1-yl (U), Uracil-5-yl (*ψ*), Hypoxanthin-9-yl (I), Thymin-1-yl (T) und 2-Aminoadenin-9-yl ausgewählt ist,
V bei jedem Nukleotid unabhängig eine 0- oder eine CH₂-Gruppe ist,
X und W jeweils in einem Nukleotid gleich oder verschieden sein können und unabhängig voneinander 0-, S-, NH₂-, Alkyl- oder Alkoxygruppen mit 1 bis 10, vorzugsweise mit 1 bis 4 Kohlenstoffatomen sind,
R Wasserstoff oder eine geradkettige oder verzweigte, gegebenenfalls mit Halogen-, Cyano-, Isocyano-, Nitro-, Amino-, Carboxyl-, Hydroxyl- oder/und Mercaptorgruppen substituierte Alkyl-, Alkenyl- oder Alkinylgruppe mit 1 bis 10 Kohlenstoffatomen ist,
und worin bei den Nukleotiden N₁, N₄, N₈ und N₁₁ in Formel (II) der Rest R in Formel (I) Wasserstoff ist und bei mindestens einem der Nukleotide N₂, N₃, N₅, N₆, N₇, N₉, N₁₀, N₁₂, N₁₃ und N₁₄ in Formel (II) der Rest R in Formel (I) von Wasserstoff verschieden ist.

B kann eine beliebige Purin- oder Pyrimidinnukleosidbase darstellen. Beispiele für geeignete Purinnukleosidbasen sind etwa Adenin-9-yl, Guanin-9-yl, Hypoxanthin-9-yl und 2-Aminoadenin-9-yl. Beispiele für Pyrimidinnukleosidbasen sind etwa Cytosin-1-yl, Uracil-1-yl, Uracil-5-yl und Thymin-1-yl.

V ist bei jedem Nukleotid unabhängig eine 0- oder eine CH₂-Gruppe, vorzugsweise eine O-Gruppe. X und W können in einem Nukleotid gleich oder verschieden sein und unabhängig voneinander 0-, S-, NH₂-, Alkyl- oder Alkoxygruppen mit 1 bis 10, vorzugsweise mit 1 bis 4 Kohlenstoffatomen bedeuten. Besonders bevorzugt sind X und W jeweils O-Gruppen (wobei in diesem Fall ein O-Atom über eine Doppelbindung an ein Phosphor gebunden wäre und das andere über eine Einfachbindung gebunden wäre und eine negative Ladung aufweisen würde).

R ist Wasserstoff oder eine geradkettige oder verzweigte, gegebenenfalls mit Halogen (Fluor, Chlor, Brom, Jod), Cyano-, Isocyano-, Nitro-, Amino-, Carboxyl-, Hydroxyl- oder/und Mercaptogruppen substituierte Alkyl-, Alkenyl- oder Alkinylgruppen mit 1 bis 10 Kohlenstoffatomen. Die von Wasserstoff verschiedenen Reste R enthalten vorzugsweise 1 bis 6 Kohlenstoffatome. Beispiele für bevorzugte Reste R sind Methyl-, Ethyl-, Propyl-, Allyl-, Dimethylallyl-, Butyl- oder Cyanomethylreste. Besonders bevorzugt ist R ein Alkenylrest mit 1 bis 4 Kohlenstoffatomen, z.B. ein Allylrest.

Eine erfindungsgemäße katalytische Oligonukleotidstruktur ist dadurch gekennzeichnet, daß bei mindestens einem der Nukleotide der Rest R in Formel (I) von Wasserstoff verschieden ist. Dabei ist es bevorzugt, wenn die Oligonukleotidstruktur eine möglichst geringe Anzahl von Nukleotiden enthält, in denen R Wasserstoff bedeutet. Derartige Oligonukleotide weisen eine hohe Resistenz gegenüber Nukleasen auf und zeigen geringere Wechselwirkungen mit Nukleinsäure-bindenden Proteinen. Andererseits darf nicht bei allen Nukleotiden der Rest R von Wasserstoff verschieden sein, da ansonsten das Oligonukleotid keine katalytische Aktivität mehr aufweist. Insbesondere innerhalb des katalytisch aktiven Zentrums des erfindungsgemäßen Oligonukleotids existieren einzelne Nukleotide, in denen der Rest R ein Wasserstoffatom darstellt.

Das aktive Zentrum einer katalytischen RNA besitzt eine sogenannte Hammerkopf-Struktur (Hotchins et al., Nucleic Acids Res. 14 (1986), 3627; Kiese und Symons in: Viroids and viroid-like pathogens, J.S. Semancik, Herausgeber (CRC-Press, Bocaratan, Florida (1987), S. 1-47). Das katalytische Zentrum der Hammerkopfstruktur enthält 3 Stämme und kann durch benachbarte Sequenzbereiche der RNA oder aber auch durch Bereiche gebildet werden, die durch viele Nukleotide voneinander getrennt sind.

Gegenstand der vorliegenden Erfindung ist daher eine Hammerkopf-Oligonukleotidstruktur mit der allgemeinen Strukturformel (II): 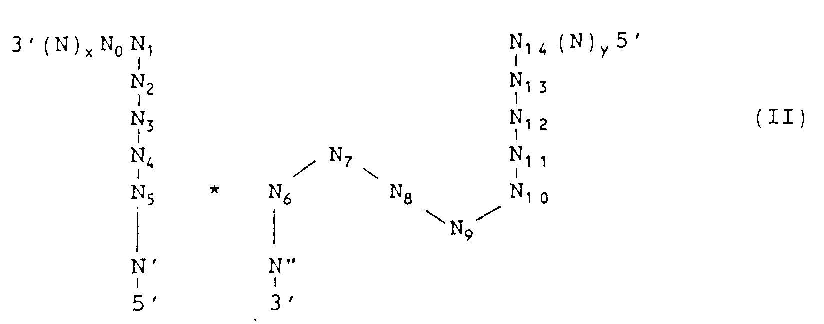 worin
N jeweils ein Nukleotid gemäß der allgemeinen Struktur-formel (I) darstellt,
x und y gleich oder verschieden sein können und x ≥ 1 und y ≥ 2 ist,
N₅ und N₆ zueinander jeweils komplementäre Nukleotide sind und ∗ eine Basenpaarung darstellt,
N' und N" entweder zwei Nukleotidsequenzen darstellen, die mindestens teilweise zueinander komplementäre Nukleotide enthalten, so daß eine stabile Basenpaarung zwischen den beiden Nukleotidsequenzen ermöglicht wird, oder
N' und N" zusammen eine einzige Nukleotidsequenz darstellen, wobei mindestens ein Teil der Sequenz durch Basenpaarung zwischen komplementären Nukleotiden einen doppelsträngigen Stamm bilden kann,
und worin gegebenenfalls ein oder mehrere zusätzliche Nukleotide N nach N₇ oder/und N₉ insertiert werden können;
welche dadurch gekennzeichnet ist, daß bei mindestens einem der Nukleotide N₂, N₃, N₅, N₆, N₇, N₉, N₁₀, N₁₂, N₁₃ und N₁₄ in Formel (II) der Rest R in Formel (I) von Wasserstoff verschieden ist.

Der Bereich N₁ bis N₁₄ enthält das katalytische Zentrum der Oligonukleotidstruktur (II). Die mit (N)ₓ und (N)_{y} bezeichneten Nukleotide befinden sich außerhalb des aktiven Zentrums und enthalten Bereiche, die für die Hybridisierung mit einer spezifischen Nukleinsäure-Zielsequenz verantwortlich sind. Die Länge dieser Bereiche ist so, daß x ≥ 1 und y ≥ 2 sein muß. Vorzugsweise sind x und y ≤ 20, größere Werte für x oder/und y bringen keine spezifischen Vorteile, erschweren aber die Synthese des Oligonukleotids. Die Oligonukleotidstruktur II kann ein entweder zusammenhängendes Molekül sein oder aus zwei verschiedenen Molekülen bestehen, d.h. N' und N" stellen entweder zusammen eine einzige Nukleotidsequenz oder zwei verschiedene Nukleotidsequenzen dar. Für die erfindungsgemäße Struktur ist wesentlich, daß die Nukleotidsequenzen N' und N" mindestens teilweise zueinander komplementäre Bereiche enthalten, die eine stabile Basenpaarung zwischen beiden Nukleotidsequenzen ermöglichen. Unter dem Begriff einer stabilen Basenpaarung ist dabei zu verstehen, daß die Oligonukleotidstruktur unter physiologischen Bedingungen bei Raumtemperatur und vorzugsweise bei Temperaturen bis zu 40°C als doppelsträngiger Strang vorliegt.

Die erfindungsgemäße Oligonukleotidstruktur zeichnet sich aus, daß bei mindestens einem, vorzugsweise bei mehreren der Nukleotide N₂, N₃, N₅ N₆, N₇, N₉, N₁₀, N₁₂, N₁₃ und N₁₄ der Rest R in Formel (I) von Wasserstoff verschieden ist. Bevorzugt ist, wenn die Nukleosidbase bei N₁ Adenin-1-yl oder 2-Aminoadenin-9-yl und bei den Nukleotiden N₄, N₈ und N₁₁ jeweils Guanin-1-yl (oder Hypoxanthin-9-yl) ist.

Ein besonders bevorzugter Gegenstand der vorliegenden Erfindung ist eine Oligonukleotidstruktur mit der allgemeinen Strukturformel (III): 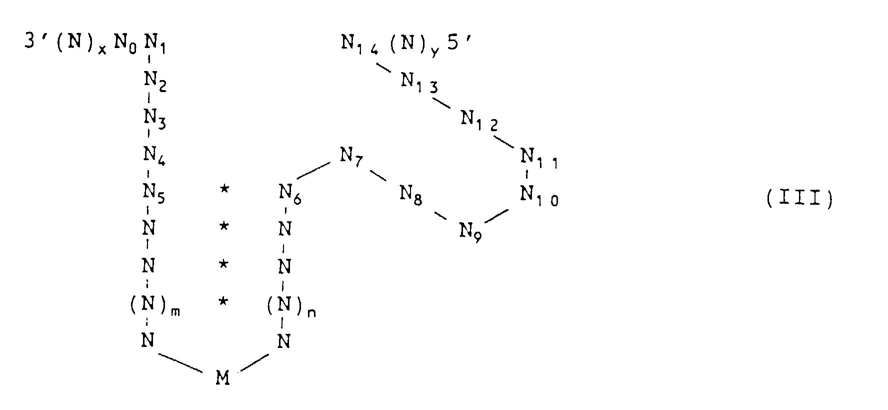 worin
N, x und y wie in Anspruch 3 definiert sind,
M eine chemische Bindung darstellt oder eine Nukleotidsequenz (N)ₐ bedeutet, wobei a ≥ 1 ist, m und n gleich oder verschieden sind und gegebenenfalls ein oder mehrere zusätzliche Nukleotide nach N₇ oder/und N₉ insertiert werden können.

Die Reste R in Formel (III) bei den Nukleotiden N₁, N₄, N₈, N₁₀, N₁₁ und N₁₂ sind vorzugsweise Wasserstoff. Die Reste R in Formel (III) bei allen Nukleotiden außer N₁, N₄, N₈, N₁₀, N₁₁ und N₁₂ sind vorzugsweise von Wasserstoff verschieden.

Ein bevorzugtes konkretes Beispiel für ein erfindungsgemäßes Oligonukleotid mit einer Hammerkopfstruktur als katalytisches Zentrum besitzt eine Struktur gemäß Formel (II) oder (III) und ist dadurch gekennzeichnet, daß die Reste V, W und X in Formel (I) O-Gruppen sind und daß bei den Nukleotiden N₁ bis N₁₄ in Formel (I) oder (III) die Reste B und R die folgenden Bedeutungen besitzen:
- N₁ :: B = A und R = H,
- N₂ :: B = A und R = Allyl,
- N₃ :: B = A und R = Allyl,
- N₄ :: B = G und R = H,
- N₅ :: B = C und R = Allyl,
- N₆ :: B = G und R = Allyl,
- N₇ :: B = A und R = Allyl,
- N₈:: B = G und R = H,
- N₉:: B = U und R = Allyl,
- N₁₀:: B = A und R = H,
- N₁₁:: B = G und R = H,
- N₁₂:: B = U und R = H,
- N₁₃ :: B = C und R = Allyl,
- N₁₄ :: B = U und R = Allyl.

Ein weiteres bevorzugtes konkretes Beispiel für ein Oligonukleotid mit einer Hammerkopfstruktur als katalytisches Zentrum ist dadurch gekennzeichnet, daß die Reste V, W und X in Formel (I) O-Gruppen sind mit der Ausnahme, daß die Verbindung zwischen N₁₁ und N₁₂ eine Phosphorthioatgruppe (X = 0 und W = S) ist, und daß bei den Nukleotiden N₁ bis N₁₄ in Formel (I) oder (III) die Reste B und R die obigen Bedeutungen besitzen.

Ein weiteres beispielhaftes Oligonukleotid ist dadurch gekennzeichnet, daß die Reste V, W und X in Formel (I) 0-Gruppen sind und daß bei den Nukleotiden N₁ bis N₁₄ in Formel (I) oder (III) die Reste B die obige Bedeutung besitzen und daß für N₁, N₄, N₈, N₁₀, N₁₁ und N₁₂ R = H, für N₉ R = 3,3-Dimethylallyl und für N₂, N₃, N₅, N₆, N₇, N₁₃ und N₁₄ R = Allyl ist. Ein weiteres Oligonukleotid unterscheidet sich von der letztgenannten Struktur nur dadurch, daß für N₃ R = 3,3-Dimethylallyl und für N₉ R = Allyl ist.

In noch einer weiteren Struktur ist für N₁, N₄, N₈, N₁₀, N₁₁ und N₁₂ R = H, für N₂, N₃, N₇, N₉ und N₁₃ R = Cyanomethyl und für N₅ N₆ und N₁₄ R = Allyl, wobei die Reste B die oben angegebenen konkreten Bedeutungen besitzen.

Weiterhin kann es auch bevorzugt sein, daß bei den erfindungsgemäßen Oligonukleotidstrukturen einer oder mehrere der Reste R = Butyl sind, um die Aufnahme der katalytischen Strukturen durch die Zelle zur verbessern. Ein konkretes Beispiel hierfür ist ein Oligonukleotid, bei dem für N₁, N₄, N₈, N₁₀, N₁₁ und N₁₂ R = H, für N₅ und N₆ R = Butyl und für N₂, N₃, N₇, N₉, N₁₃ und N₁₄ R = Allyl ist, wobei gegebenenfalls für einen, mehrere oder alle der Reste N, die in Formel (III) in basengepaarter Form (durch einen Stern gekennzeichnet) vorliegen, R ebenfalls Butyl sein kann.

Zur zusätzlichen Stabilisierung der erfindungsgemäßen Oligonukleotidstrukturen können sich an ihrem freien 3'-Ende bzw. an ihren freien 3'-Enden 3'-Deoxyribonukleotide oder/und 3'-O-Alkylribonukleotide befinden. Auf diese Weise wird das erfindungsgemäße Oligonukleotid vor einem 3'-Exonukleaseabbau geschützt.

Die erfindungsgemäßen Oligonukleotide können weiterhin zur Stabilisierung ihrer räumlichen Konfiguration Nukleotide enthalten, deren Nukleosidbasen durch ein Quervernetzungsmittel modifiziert sind. Ein Beispiel für ein derartiges Quervernetzungsmittel ist Psoralen oder ein Psoralenderivat. Auf diese Weise können doppelsträngige Oligonukleotidstrukturen durch kovalente Quervernetzung modifizeirt werden. Die Herstellung von Nukleotiden, die mit einem Quervernetzungsmittel modifiziert sind, ist detailliert in der DE-A 39 28 900 offenbart.

Ferner kann die erfindungsgemäße Oligonukleotidstruktur mit einer prostethischen Gruppe verknüpft sein, um die Aufnahme in der Zelle oder/und die spezifische zelluläre Lokalisierung der Oligonukleotidstruktur zu verbessern. Beispiele für derartige prostethische Gruppen sind Polyaminosäuren (z.B. Polylysin), Lipide, Hormone oder Peptide. Die Verknüpfung dieser prostethischen Gruppen erfolgt üblicherweise über freie 5'-bzw. 3'-Enden der erfindungsgemäßen Oligonukleotidstruktur, wobei diese Verknüpfung entweder direkt oder über einen Linker erfolgen kann. Beispiele für Linker sind etwa am terminalen 5'-Phosphat vorliegende Diester mit Amino- oder Phosphatfunktion oder mit Merkaptoalkoxygruppen.

Die Synthese der erfindungsgemäßen Oligonukleotidstrukturen erfolgt auf an sich bekannte Weise aus Monomereinheiten. Derartige Monomereinheiten besitzen üblicherweise die allgemeine Formel (V) 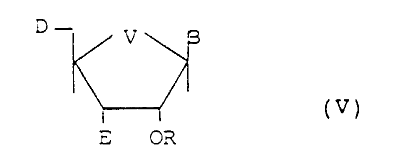 worin
V, B und R wie in Formel (I) definiert sind und
D und E zur Ausbildung von 3'- bis 5'-Internukleotidbindungen fähige reaktive Gruppen bedeuten. Solche Gruppen sind dem Fachmann bekannt und z.B. in B. Sproat et al., Nucleic Acids Res. 18 (1990), 41-49 sowie zusammenfassend in E.L. Winnacker, Gene und Klone, VCH-Verlagsgesellschaft mbH, Weinheim (Deutschland) (1985), insbesondere Seiten 44-49 und in Froehler und Matteucci, Tetrahedron Let. (1986), S. 469-472 beschrieben. Mit reaktiven Mononukleotiden der Formel (V) lassen sich auf bekannte Weise, insbesondere an einer festen Phase die erfindungsgemäßen Oligonukleotidstrukturen herstellen.

Ein weiterer Gegenstand der vorliegenden Erfindung ist ein Verfahren zur Spaltung einer Nukleinsäure-Zielsequenz unter Verwendung einer synthetischen katalytischen Oligonukleotidstruktur gemäß vorliegender Erfindung. Diese Nukleinsäure-Zielsequenz kann dabei entweder ein Teil der synthetischen katalytischen Oligonukleotidstruktur selbst sein, oder es kann sich bei dieser Nukleinsäure-Zielsequenz um ein von der synthetischen katalytischen Oligonukleotidstruktur verschiedenes Molekül handeln.

Verwendet man zur Spaltung einer Nukleinsäure-Zielsequenz ein erfindungsgemäßes Oligonukleotid mit einer Hammerkopfstruktur der allgemeinen Formel (II), so wird üblicherweise eine Zwischenstufe mit der folgenden Struktur ausgebildet: 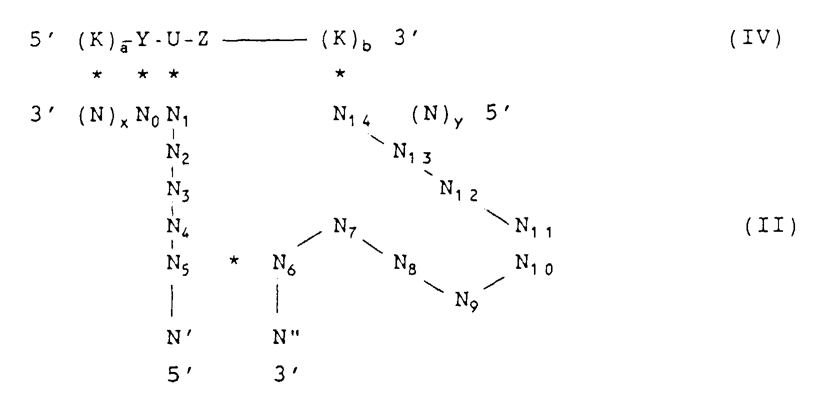 wobei
die Symbole N, N', N'', x, y und * wie in Anspruch 3 definiert sind,
und K, Y, U und Z Nukleotide der Nukleinsäure-Zielsequenz darstellen,
worin U Uridin ist,
Z ein nicht modifiziertes Ribonukleotid, ausgewählt aus der Gruppe, bestehend aus Adenosin, Cytidin oder Uridin ist,
K und Y beliebige Nukleotide sind,
a ≥ 1 und b ≥ 3 ist,
und die Spaltung der Nukleinsäure-Zielsequenz 3'-seitig der Nukleotidsequenz YUZ erfolgt, und wobei gegebenenfalls eine chemische Bindung zwischen dem 5'-Ende der Nukleinsäure-Zielsequenz (IV) und dem 3'-Ende des Olignukleotids (II) bei (N)ₓ oder zwischen dem 3'-Ende der Nukleinsäure-Zielsequenz (IV) und dem 5'-Ende des Oligonukleotids (II) bei (N)_{y} existiert.

Das Nukleotid Y in der Nukleinsäure-Zielsequenz stellt vorzugsweise einen Guanosinrest dar und Z bedeutet vorzugsweise Adenosin oder Cytidin. Die Nukleinsäure-Zielsequenz kann ein beliebiges Oligo- oder Polynukleotid sein, vorausgesetzt daß Z ein nicht-modifiziertes Ribonukleotid ist. Der Rest der Zielsequenz kann beispielsweise DNA, 2'-O-Alkyl-RNA oder eine gemischte Struktur sein. Vorzugsweise ist die Nukleinsäure-Zielsequenz jedoch eine RNA. Die Spaltungsspezifität des erfindungsgemäßen Oligonukleotids für eine bestimmte Nukleinsäure-Zielsequenz kann dadurch erreicht werden, indem die Sequenz der Hybridisierungsarme der katalytischen Komponente (N₀ (N)X bzw. (N)YN₁₄) so verändert werden, daß sie zu den Sequenzen komplementär sind, welche die Spaltstelle der gewünschten Zielsequenz flankieren.

Das erfindungsgemäße Verfahren kann sowohl innerhalb einer lebenden Zelle als auch in vitro durchgeführt werden. Vorzugsweise wird die Spaltung in Gegenwart eines divalenten Kations, insbesondere Mg²⁺ und bei einem pH-Wert von etwa 7 bis 9, besonders bevorzugt etwa 8 durchgeführt.

Ein weiterer Gegenstand der vorliegenden Erfindung ist die Verwendung einer erfindungsgemäßen Oligonukleotidstruktur zur katalytischen Spaltung einer Nukleinsäure-Zielsequenz, wobei die Nukleinsäure-Zielsequenz entweder ein Teil der katalytischen Oligonukleotidstruktur ist oder ein davon verschiedenes Molekül darstellt.

Ferner betrifft die Erfindung ein Arzneimittel, das als Wirkstoff eine erfindungsgemäße Oligonukleotidstruktur, gegebenenfalls zusammen mit üblichen pharmazeutischen Träger-, Hilfs-, Füll- oder/und Verdünnungsmitteln enthält. Die Erfindung betrifft auch ein Verfahren zur Herstellung eines Arzneimittels für die antivirale Therapie in Menschen, Tieren und Pflanzen, wobei man als Wirkstoff eine erfindungsgemäße Oligonukleotidstruktur verwendet. Ein Beispiel für eine derartige antivirale Therapie wäre die Bekämpfung von AIDS mit den erfindungsgemäßen Oligonukleotiden (siehe z.B. Sarver et al., Science 247 (1990), 1222-1225).

Ein weiterer Gegenstand der vorliegenden Erfindung ist auch ein diagnostisches Reagenz, das als Bestandteil eine erfindungsgemäße Oligonukleotidstruktur enthält sowie ein Verfahren zur Herstellung eines derartigen diagnostischen Reagenz. Dieses erfindungsgemäße diagnostische Reagenz kann beispielsweise für eine genetische Musterungsprozedur verwendet werden. Die Erfindung soll weiterhin durch die folgenden Beispiele und die Sequenzprotokolle SEQ ID N0.1 und SEQ ID N0.2 verdeutlicht werden.

Es zeigen:
- SEQ ID N0.1:: die Nukleotidsequenz einer Substrat-RNA und
- SEQ ID N0.2:: die Nukleotidsequenz einer katalytisch aktiven Ribozym-RNA

### Beispiel 1

### Oligonukleotidsynthese, Deblockierung und Reinigung

Die Synthese von 2'-O-Methylribonukleosid-3'-O-phosphoramidit-Bausteinen erfolgte nach Sproat et al. (Nucleic Acids Res. 18 (1990), 41-49). 2'-O-Allylribonukleosid-3'-O-phosphoramidit-Bausteine wurden nach Sproat et al. (Nucleic Acids Res. 19 (1991), 733-738) synthetisiert.

2'-0-[1-(2-Fluorphenyl)-4-methoxypiperidin-4-yl]ribonukleosid-3'-0-phophoramidit-Bausteine wurden nach Beijer et al. (Nucleic Acids Res. 18 (1990), 5143-5151) synthetisiert.

Die Oligonukleotide wurden nach dem β-Cyanoethylphosphoramidit-Verfahren auf Controlled Pore Glass unter Verwendung eines modifizierten DNA-Synthesezyklus auf einen Applied Biosystems Synthetisierapparat zusammengesetzt. Anstelle von Tetrazol wurde 5-(4-Nitrophenyl)-1H-Tetrazol als Aktivator für den Kondensationsschritt verwendet, wobei die Reaktionsdauer für die Kondensation auf 12 Minuten erhöht wurde (vgl. Nucleic Acids Res. 18 (1990), 41-49 und 5141-5151).

Die Deblockierung und Reinigung wurde durchgeführt, indem ein Träger, an dem ein völlig blockiertes Oligonukleotid gebunden war, zunächst mit einer Lösung von 25 %igem wäßrigem Ammoniak in einem abgedichteten sterilen Gefäß für 10 Stunden bei 60°C behandelt wurde. Die abgekühlte Lösung wurde dann in einem sterilen Gefäß im Vakuum bis zur Trockene eingedampft. Das Oligonukleotid-Rohprodukt, welches noch eine 5'-terminale 5'-O-Dimethoxytrithyl-Schutzgruppe und einige 2'-O-Fpmp-Schutzgruppen enthält, wurde dann durch Reverse Phase HPLC auf einer µ-Bondapak C₁₈-Säule unter Verwendung eines Acetonitrilgradienten in wäßrigem 0,1 mol/l Triethylammoniumacetat pH 7,0 als Elutionsmittel gereinigt. Der Produktpeak wurde gesammelt und das Lösungsmittel im Vakuum entfernt. Der Rückstand wurde in 1 ml 10 %igem Glycerin in Wasser resuspendiert und die Lösung wurde 10 Minuten zentrifugiert. Der Überstand wurde auf eine G15 Sephadex-Säule (30 ml) gegeben, die mit sterilem destilliertem Wasser eluiert wurde. Das Leervolumen der Säule (etwa 9 ml) wurde verworfen und eine 3 ml-Fraktion wurde gesammelt, welche das teilweise blockierte Oligonukleotid enthielt. Dann wurde sterile wäßrige Salzsäure (270 µl, 0,1 mol/l) zugegeben, wodurch ein pH im Bereich von 2 bis 2,5 erhalten wurde. Die Lösung wurde 20 Stunden lang bei 20 bis 25°C gehalten, um die säurelabilen DMTr- und Fpmp-Schutzgruppen zu entfernen. Anschließend wurde die Lösung 10 Minuten bei 2000 Upm zentrifugiert. Der Überstand wurde durch Zugabe von 2 mol/l Tris Acetat (75 µl, pH 7,9) neutralisiert. Das reine vollständig entblockierte Oligonukleotid wurde aus der wäßrigen Lösung unter Verwendung von wiederholten Extraktionen mit 1-Butanol gewonnen, wie bei Cathala und Brunel (Nucleic Acids Res. 18 (1990), 201) beschrieben. Das Oligonukleotidpellet wurde getrocknet und in 100 µl Tris-Puffer (10 mmol/l, pH 7,5, 1 mmol/l EDTA) gelöst.

### Beispiel 2

### Herstellung und Markierung von Substrat-RNA

Chemisch synthetisierte Substratoligonukleotide wurden entweder mit γ-³²P-ATP und Kinase oder mit RNA-Ligase und ³²P-pCp gemäß den von den Herstellern der jeweiligen Enzyme vorgeschlagenen Standardprozeduren markiert.

Alternativ dazu wurde RNA auch durch Transkription mit SP6-RNA-Polymerase nach Linearisierung der jeweiligen Matrizen mit geeigneten Restriktionsenzymen synthetisiert. Die RNA-Moleküle wurden durch Einbau von α-³²P-UTP in das Transkript markiert. Die Bedingungen der Transkription waren wie folgt:
40 mmol/l Tris, pH 7,5
6 mmol/l MgCl₂
2 mmol/l Spermidin
10 mmol/l Dithiotreitol
500 mmol/l Nukleosidtriphosphate (A, C, G)
100 µmol/l UTP
10 µCi α-³²P-UTP
10 U/µl SP6-Polymerase
20 U/µl RNAse-Inhibitor (menschliche Plazenta)

Nach zweistündiger Inkubation bei 37°C wurde die als Matrize verwendete DNA mit RNAse-freier DNAse verdaut. RNA wurde durch Gelelektrophorese auf 6 %igen Polyacrylamidgelen (1:30 Acrylamid:Bisacrylamid) in Gegenwart von 7 mol/l Harnstoff aufgetrennt. RNA-Banden wurden durch Diffusion eluiert und RNA wurde durch Ethanolpräzipitation gewonnen. Die Aktivitätsbestimmung wurde in einer 10 µl Reaktionsmischung ausgeführt, welche Substrat RNA (10 nM bis 1 µM), Tris 50 mM pH 7,5, MgCl₂ 20 mM, 0,01 bis 10 pMol Ribozym enthält. Diese Mischung wurde bei 50°C 60 Minuten inkubiert. Die Spaltprodukte wurden auf 7 % Polyacrylamidgel in Gegenwart von 7 M Harnstoff aufgetrennt.

### Beispiel 3

Bestimmung der Aktivität unterschiedlicher, modifizierter, katalytischer Oligonukleotide.

Als Zielsequenz wurde ein 17 Nukleotide langes, chemisch synthetisiertes Oligoribonukleotid, basierend auf dem EDB Exon menschlicher Fibronektin-mRNA, verwendet. Die Sequenz (SEQ ID NO.1) war wie folgt:

5'- r[UACACAGUCACAGGGCU]

Das zur Spaltung dieser Sequenz verwendete Ribozym besaß die im folgenden dargestellte Nukleotidsequenz (SEQ ID NO.2):

5'- r[GCCCUGUCUGAUGAGUCCGUGAGGACGAAACUGUGU]

Diese Sequenz ist als E0 bezeichnet. Die unterstrichenen Bereiche bezeichnen die Nukleotide N₁₄ bis N₆ bzw. N₅ bis N₀ gemäß Formel (III). Weiterhin wurden die folgenden Analoga von E0 (mit einer identischen Basenzusammensetzung) synthetisiert:
E1: R = H für N₁, N₄, N₈, N₁₀, N₁₁ und N₁₂ ; R = Allyl für alle anderen N.
E2: R = H für N₁ , N₄, N₈, N₁₀ und N₁₁ ; R = Allyl für alle anderen N.
E3: R = H für N₁, N₄, N₈, N₁₀, N₁₁ und N₁₂ ; die Verbindung zwischen N₁₁ und N₁₂ ist eine Phosphorthioatgruppe (d.h. X = O und W = S gemäß Formel (I)); R = Allyl für alle anderen N.
E4: R = H für N₁, N₄, N₈, N₁₀, N₁₁ und N₁₂ ; R = 3,3-Dimethylallyl für N₉ und R = Allyl für alle anderen N.
E5: R = H für N₁, N₄, N₈, N₁₀, N₁₁ und N₁₂; R = 3,3-Dimethylallyl für N₃ und R = Allyl für alle anderen N.
E6: R = H für N₁, N₄, N₈, N₁₀, N₁₁ und N₁₂; R = Butyl für N₅ und N₆ sowie für die anderen 6 N (UCC und GGA), die einen basengepaarten Bereich bilden. Für alle anderen N ist R = Allyl.
E7: R = H für N₁, N₄, N₈, N₁₀, N₁₁ und N₁₂ ; R = Cyanomethyl für N₂, N₃, N₇, N₉ und N₁₃ ; R = Allyl für alle restlichen N.

Der Test zur Bestimmung der Spaltaktivität wurde in einem Reaktionsvolumen von 10 µl durchgeführt, das Substrat RNA (10 nmol/l bis 1 µmol/l), Tris 50 mmol/l pH 7,5, MgCl₂ 20 mmol/l und 0,01 bis 10 pmol Ribozym enthielt. Das Reaktionsgemisch wurde 60 Minuten lang bei 50°C inkubiert. Die Spaltprodukte wurden durch Polyacrylamid-Gelelektrophorese in Gegenwart von 7 mol/l Harnstoff aufgetrennt.

Die Reaktionskinetik wurde durch Messung der Menge an ³²P-radioaktiv markiertem Substrat bei einer Spaltungsdauer von 1,5, 10 und 15 Minuten bei 50°C bestimmt. Die Substratkonzentrationen waren wie folgt: 25, 50, 100, 250 nmol/l; die Ribozymkonzentration war zwischen 4 nmol/l und 20 nmol/l. Die Proben wurden vorgewärmt und die Reaktion wurde durch Zugabe von Ribozym gestartet. Nach der vorbestimmten Reaktionsdauer wurde die Reaktion durch Zugabe 2 Volumina 20 mmol/l EDTA gestoppt. Die Produkte wurden durch Polyacrylamid-Harnstoff-Gelelektrophorese aufgetrennt und mit dem Molecular Dynamics Phosphor Imager quantitativ analysiert.

In der folgenden Tabelle 1 sind die relative Aktivität und die RNase-Sensitivität der oben aufgelisteten katalytischen Oligonukleotide E0, E1, E2, E3, E4, E5, E6 und E7 angegeben.

**Tabelle 1**

| Katalytische Struktur | Relative Aktivität | RNase A Sensitifität |
|---|---|---|
| E0 | 1 | 1 |
| E1 | 0,2 | 0,01 |
| E2 | < 0,05 | stabil |
| E3 | 0,1 | 0,001 |
| E4 | ca. 0,2 | 0,01 |
| E5 | ca. 0,2 | 0,01 |
| E6 | ca. 0,2 | 0,01 |
| E7 | ca. 0,2 | 0,01 |

### SEQ ID NO.1:

Nukleinsäure (RNA):
einzelsträngig
linear
Länge: 17

UACACAGUCA CAGGGCU 17

### SEQ ID NO.2:

Nukleinsäure (RNA):
einzelsträngig
linear
Länge: 36

GCCCUGUCUG AUGAGUCCGU GAGGACGAAA CUGUGU 36

## Patentansprüche (Patentansprüche für folgende(n) Vertragsstaat(en): AT, BE, CH, LI, DE, FR, GB, IT, LU, MC, NL, PT, SE)

1. Synthetische katalytische Oligonukleotidstruktur, die zur Hybridisierung an eine und zur Spaltung einer Nukleinsäure-Zielsequenz geeignet ist und eine Oligonukleotidstruktur mit der allgemeinen Strukturformel (II) hat: 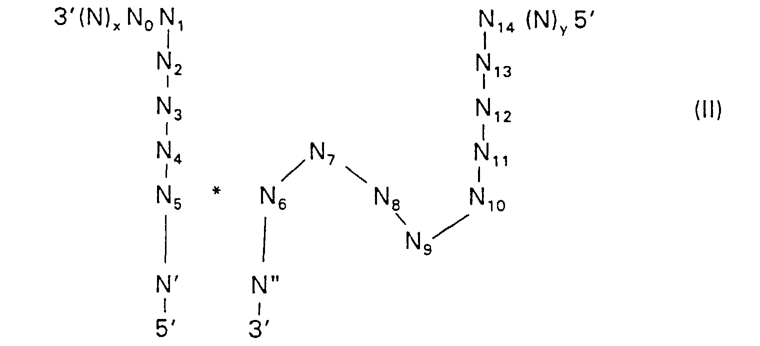 worin
N jeweils ein Nukleotid gemäß der allgemeinen Strukturformel (I) darstellt,
x und y gleich oder verschieden sein können und x ≥ 1 und y ≥ 2 ist, N₅ und N₆ zueinander jeweils komplementäre Nukleotide sind und * eine Basenpaarung darstellt,
N' und N" entweder zwei Nukleotidsequenzen darstellen, die mindestens teilweise zueinander komplementäre Nukleotide enthalten, so daß eine stabile Basenpaarung zwischen den beiden Nukleotidsequenzen ermöglicht wird,
oder
N' und N" zusammen eine einzige Nukleotidsequenz darstellen, wobei mindestens ein Teil der Sequenz durch Basenpaarung zwischen komplementären Nukleotiden einen doppelsträngigen Stamm bilden kann,
und worin gegebenenfalls ein oder mehrere zusätzliche Nukleotide N nach N₇ oder/und N₉ insertiert werden können,
und worin die Oligonukleotidstruktur Nukleotide mit der allgemeinen Strukturformel (I) enthält: 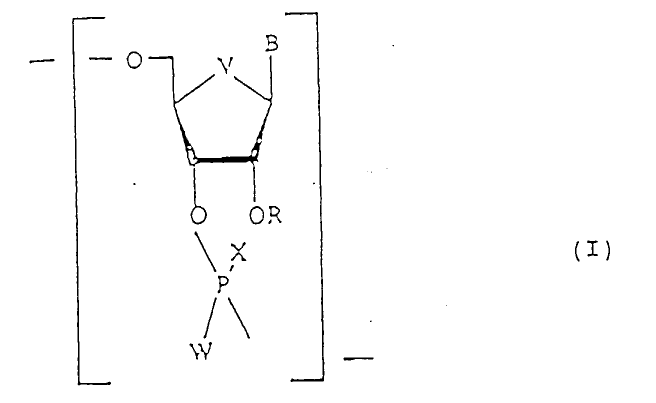
worin
B eine Nukleosidbase darstellt, die insbesondere aus der Gruppe, bestehend aus Adenin-9-yl (A), Cytosin-1-yl (C), Guanin-9-yl (G), Uracil-1-yl (U), Uracil-5-yl (ψ), Hypoxanthin-9-yl (I), Thymin-1-yl (T) und 2-Aminoadenin-9-yl ausgewählt ist,
V bei jedem Nukleotid unabhängig eine O- oder ein CH₂-Gruppe ist,
X und W jeweils in einem Nukleotid gleich oder verschieden sein können und unabhängig voneinander O-, S-, NH₂-, Alkyl- oder Alkoxygruppen mit 1 bis 10, vorzugsweise mit 1 bis 4 Kohlenstoffatomen sind,
R Wasserstoff oder eine geradkettige oder verzweigte, gegebenenfalls mit Halogen-, Cyano-, Isocyano-, Nitro-, Amino-, Carboxyl-, Hydroxyl- oder/und Mercaptogruppen substituierte Alkyl-, Alkenyl- oder Alkinylgruppe mit 1 bis 10 Kohlenstoffatomen ist,
und worin bei den Nukleotiden N₁, N₄, N₈ und N₁₁ in Formel (II) der Rest R in Formel (I) Wasserstoff ist und bei mindestens einem der Nukleotide N₂, N₃, N₅, N₆, N₇, N₉, N₁₀, N₁₂, N₁₃ und N₁₄ in Formel (II) der Rest R in Formel (I) von Wasserstoff verschieden ist.

2. Oligonukleotidstruktur nach Anspruch 1,
**dadurch gekennzeichnet,**
daß die Reste B in Formel (I) bei den Nukleotiden N₁, N₄, N₈ bzw. N₁₁ jeweils A oder 2-Aminoadenin-9-yl, G, G bzw. G sind.

3. Oligonukeotidstruktur nach einem der vorhergehenden Ansprüche,
**dadurch gekennzeichnet,**
daß die Reste V, W und X in Formel (I) O-Gruppen sind.

4. Oligonukleotidstruktur nach einem der vorhergehenden Ansprüche,
**dadurch gekennzeichnet,**
daß die von Wasserstoff verschiedenen Reste R in Formel (I) 1 bis 6 Kohlenstoffatome enthalten.

5. Oligonukleotidstruktur nach Anspruch 4,
**dadurch gekennzeichet,**
daß die von Wasserstoff verschiedenen Reste R in Formel (I) aus der Gruppe, bestehend aus Methyl-, Ethyl-, Propyl-, Allyl-, Dimethylallyl-, Butyl- oder Cyanomethylresten ausgewählt sind.

6. Oligonukleotidstruktur nach einem der vorhergehenden Ansprüche mit der allgemeinen Strukturformel (III): 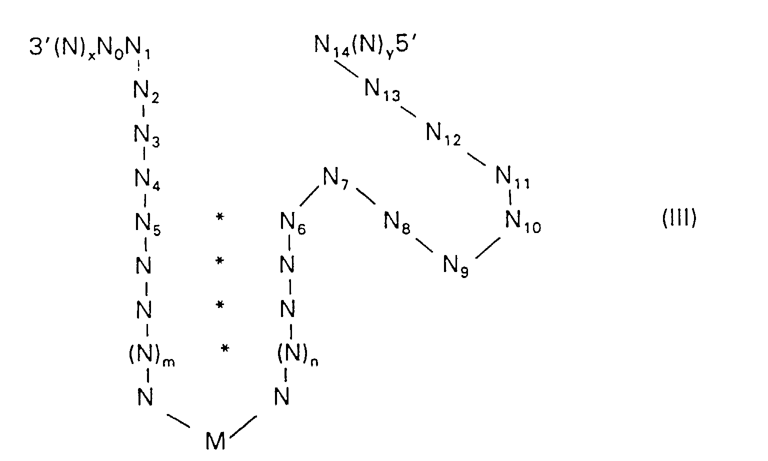 worin
N, x und y wie in Anspruch 1 definiert sind,
M eine chemische Bindung darstellt oder eine Nukleotidsequenz (N)ₐ bedeutet, wobei a ≥ 1 ist, m und n gleich oder verschieden sind und gegebenenfalls ein oder mehrere zusätzliche Nukleotide nach N₇ oder/und N₉ isertiert werden können.

7. Oligonukleotidstruktur nach Anspruch 6,
**dadurch gekennzeichnet,**
daß die Reste R in Formel (III) bei den Nukleotiden N₁, N₄, N₈, N₁₀, N₁₁ und N₁₂ Wasserstoff sind.

8. Oligonukleotidstrukur nach Anspruch 6 oder 7,
**dadurch gekennzeichnet,**
daß die Reste R in Formel (III) bei allen Nukleotiden außer N₁, N₄, N₈, N₁₀, N₁₁ und N₁₂ von Wasserstoff verschieden sind.

9. Oligonukleotidstruktur nach einem der vorhergehenden Ansprüche,
**dadurch gekennzeichnet,**
daß die Reste V, W und X in Formel (I) O-Gruppen sind und daß bei den Nukleotiden N₁ bis N₁₄ in Formel (I) oder (III) die Reste B und R die folgenden Bedeutungen besitzen:
N₁ : B = A und R = H,
N₂ : B = A und R = Allyl,
N₃ : B = A und R = Allyl,
N₄ : B = G und R = H,
N₅ : B = C und R = Allyl,
N₆ : B = G und R = Allyl,
N₇ : B = A und R = Allyl,
N₈ : B = G und R = H,
N₉ : B = U und R = Allyl,
N₁₀ : B = A und R = H,
N₁₁ : B = G und R = H,
N₁₂ : B = U und R = H,
N₁₃ : B = C und R = Allyl,
N₁₄ : B = U und R = Allyl.

10. Oligonukleotidstruktur nach einem der Ansprüche 1 bis 8,
**dadurch gekennzeichnet,**
daß die Reste V, W und X in Formel (I) O-Gruppen sind, mit der Ausnahme, daß die Verbindung zwischen N₁₁ und N₁₂ eine Phosphorthioatgruppe (X = O und W = S) ist, und daß bei den Nukleotiden N₁ bis N₁₄ in Formel (I) oder (III) die Reste B und R die Bedeutung gemäß Anspruch 9 besitzen.

11. Oligonukleotidstruktur nach einem der Ansprüche 1 bis 8,
**dadurch gekennzeichnet,**
daß die Reste V, W und X in Formel (I) 0-Gruppen sind und daß bei den Nukleotiden N₁ bis N₁₄ in Formel (I) oder (III) die Reste B die Bedeutung gemäß Anspruch 9 besitzen und daß für N₁, N₄, N₈, N₁₀, N₁₁ und N₁₂ R = H, für N₉ R = 3,3-Dimethylallyl und für N₂, N₃, N₅, N₆, N₇, N₁₃ und N₁₄ R = Allyl ist.

12. Oligonukleotidstruktur nach einem der Ansprüche 1 bis 8,
**dadurch gekennzeichnet,**
daß die Reste V, W und X in Formel (I) 0-Gruppen sind und daß bei den Nukleotiden N₁ bis N₁₄ in Formel (I) oder (III) die Reste B die Bedeutung gemäß Anspruch 9 besitzen und daß für N₁, N₄, N₈, N₁₀, N₁₁ und N₁₂ R = H, für N₃ R = 3,3-Dimethylallyl und für N₂, N₅, N₆, N₇, N₉, N₁₃ und N₁₄ R = Allyl ist.

13. Oligonukleotidstruktur nach einem der Ansprüche 1 bis 8,
**dadurch gekennzeichnet,**
daß die Reste V, W und X in Formel (I) 0-Gruppen sind und daß bei den Nukleotiden N₁ bis N₁₄ in Formel (I) oder (III) die Reste B die Bedeutung gemäß Anspruch 9 besitzen und daß für N₁, N₄, N₈, N₁₀, N₁₁ und N₁₂ R = H, für N₂, N₃, N₇, N₉ und N₁₃ R = Cyanomethyl und für N₅, N₆ und N₁₄ R = Allyl ist.

14. Oligonukleotidstruktur nach einem der Ansprüche 1 bis 8,
**dadurch gekennzeichnet,**
daß die Reste V, W und X in Formel (I) 0-Gruppen sind und daß bei den Nukleotiden N₁ bis N₁₄ in Formel (I) oder (III) die Reste B die Bedeutung gemäß Anspruch 9 besitzen und daß für N₁, N₄, N₈, N₁₀, N₁₁ und N₁₂ R = H, für N₅ und N₆ R = Butyl und für N₂, N₃, N₇, N₉, N₁₃ und N₁₄ R = Allyl ist, wobei gegebenenfalls für einen oder mehrere der Reste N, die in Formel (III) in basengepaarter Form vorliegen, R = Butyl ist.

15. Oligonukleotidstruktur nach einem der vorhergehenden Ansprüche,
**dadurch gekennzeichnet,**
daß sich an den freien 3'-Enden der Oligonukleotidstruktur 3'-Deoxyribonukleotide oder/und 3'-O-Alkylribonukleotide befinden.

16. Oligonukleotidstruktur nach einem der vorhergehenden Ansprüche,
**dadurch gekennzeichnet,**
daß sie weiterhin zur Stabilisierung ihrer räumlichen Konfiguration Nukleotide enthält, deren Nukleosidbasen durch ein Quervernetzungsmittel modifiziert sind.

17. Oligonukleotidstruktur nach Anspruch 16,
**dadurch gekennzeichnet,**
daß das Quervernetzungsmittel Psoralen oder ein Psoralenderivat ist.

18. Oligonukleotidstruktur nach einem der vorhergehenden Ansprüche,
**dadurch gekennzeichnet,**
daß sie mit einer prostethischen Gruppe, ausgewählt aus Polyaminosäuren, Lipiden, Hormonen oder Peptiden verknüpft ist, um die Aufnahme in der Zelle oder/und die spezifische zelluläre Lokalisierung der Oligonukleotidstruktur zu verbessern.

19. Verfahren zur Spaltung einer Nukleinsäure-Zielsequenz unter Verwendung einer synthetischen katalytischen Oligonukleotidstruktur nach einem der Ansprüche 1 bis 18.

20. Verfahren nach Anspruch 19,
**dadurch gekennzeichnet,**
daß die Nukleinsäure-Zielsequenz ein von der synthetischen katalytischen Oligonukleotidstruktur verschiedenes Molekül ist.

21. Verfahren nach einem der Ansprüche 19 oder 20,
**dadurch gekennzeichnet**,
daß man eine Nukleinsäure-Zielsequenz der allgemeinen Formel (IV) unter Verwendung eines Oligonukleotids mit der allgemeinen Formel (II) spaltet, wobei eine Zwischenstufe mit der folgenden Struktur ausgebildet wird, 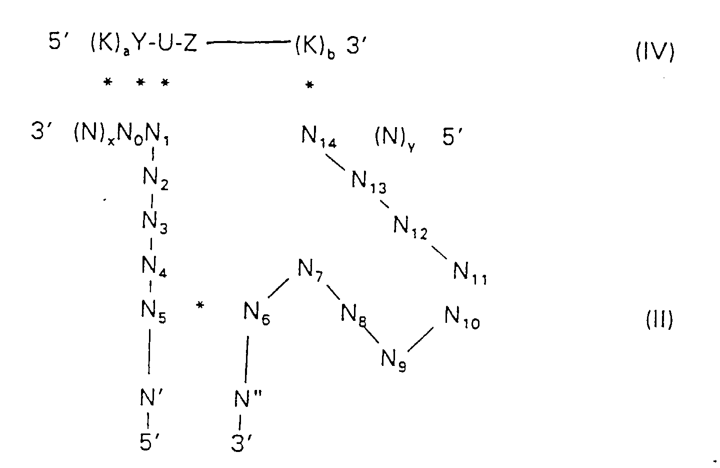 wobei
die Symbole N, N', N", x, y und * wie in Anspruch 1 definiert sind,
und K, Y, U und Z Nukleotide der Nukleinsäure-Zielsequenz darstellen, worin U Uridin ist,
Z ein nicht modifiziertes Ribonukleotid, ausgewählt aus der Gruppe, bestehend aus Adenosin, Cytidin oder Uridin ist,
K und Y beliebige Nukleotide sind,
a ≥ 1 und b ≥ 3 ist,
und die Spaltung der Nukleinsäure-Zielsequenz 3'-seitig der Nukleotidsequenz YUZ erfolgt, und wobei gegebenenfalls eine chemische Bindung zwischen dem 5'-Ende der Nukleinsäure-Zielsequenz (IV) und dem 3'-Ende des Oligonukleotids (II) bei (N)ₓ oder zwischen dem 3'-Ende der Nukleinsäure-Zielsequenz (IV) und dem 5'-Ende des Oligonukleotids (II) bei (N)_{y} existiert.

22. Verfahren nach Anspruch 21,
**dadurch gekennzeichnet,**
daß Y Guanosin ist.

23. Verfahren nach Anspruch 21 oder 22,
**dadurch gekennzeichnet,**
daß Z Adenosin oder Cytidin ist.

24. Verfahren nach einem der Ansprüche 19 bis 23,
**dadurch gekennzeichnet,**
daß die Nukleinsäure-Zielsequenz eine Ribonukleinsäure ist.

25. Verfahren nach einem der Ansprüche 19 bis 24,
**dadurch gekennzeichnet,**
daß man die Spaltung in Gegenwart eines divalenten Kations, insbesondere Mg²⁺ durchführt.

26. Verfahren nach einem der Ansprüche 19 bis 24,
**dadurch gekennzeichnet,**
daß man die Spaltung bei einem pH-Wert von etwa 7 bis 9 durchführt.

27. Verwendung einer Oligonukleotidstruktur nach einem der Ansprüche 1 bis 18 zur katalytischen Spaltung einer Nukleinsäure-Zielsequenz.

28. Arzneimittel,
**dadurch gekennzeichnet,**
daß es als Wirkstoff eine Oligonukleotidstruktur nach einem der Ansprüche 1 bis 18 gegebenenfalls zusammen mit üblichen pharmazeutischen Träger-, Hilfs-, Füll- oder/und Verdünnungsmitteln enthält.

29. Verfahren zur Herstellung eines Arzneimittels für die antivirale Therapie in Menschen, Tieren und Pflanzen,
**dadurch gekennzeichnet,**
daß man als Wirkstoff eine Oligonukleotidstruktur nach einem der Ansprüche 1 bis 18 verwendet.

30. Diagnostisches Reagenz,
**dadurch gekennzeichnet,**
daß es als Bestandteil eine Oligonukleotidstruktur nach einem der Ansprüche 1 bis 18 enthält.

31. Verfahren zur Herstellung eines diagnostischen Reagenz für eine genetische Musterungsprozedur,
**dadurch gekennzeichnet,**
daß es als Bestandteil eine Oligonukleotidstuktur nach einem der Ansprüche 1 bis 18 enthält.

## Patentansprüche (Patentansprüche für folgende(n) Vertragsstaat(en): ES)

1. Verfahren zur Herstellung von synthetischen katalytischen Oligonukleotidstrukturen aus Monomereinheiten, wobei die Oligonukleotidstruktur zur Hybridisierung an eine und zur Spaltung einer Nukleinsäure-Zielsequenz geeignet ist und eine Oligonukleotidstruktur mit der allgemeinen Strukturformel (II) hat: 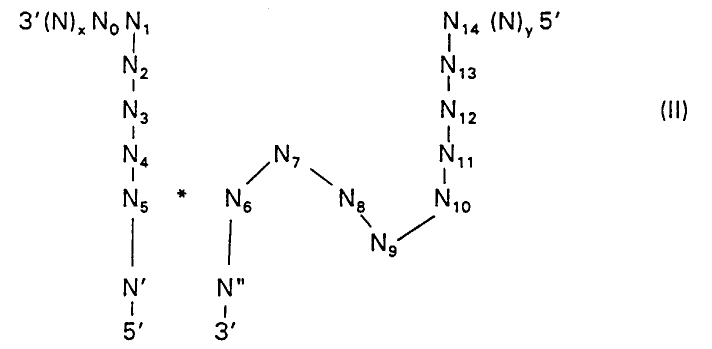 worin
N jeweils ein Nukleotid gemäß der allgemeinen Strukturformel (I) darstellt,
x und y gleich oder verschieden sein können und x ≥ 1 und y ≥ 2 ist, N₅ und N₆ zueinander jeweils komplementäre Nukleotide sind und * eine Basenpaarung darstellt,
N' und N" entweder zwei Nukleotidsequenzen darstellen, die mindestens teilweise zueinander komplementäre Nukleotide enthalten, so daß eine stabile Basenpaarung zwischen den beiden Nukleotidsequenzen ermöglicht wird,
oder
N' und N" zusammen eine einzige Nukleotidsequenz darstellen, wobei mindestens ein Teil der Sequenz durch Basenpaarung zwischen komplementären Nukleotiden einen doppelsträngigen Stamm bilden kann,
und worin gegebenenfalls ein oder mehrere zusätzliche Nukleotide N nach N₇ oder/und N₉ insertiert werden können,
und worin die Oligonukleotidstruktur Nukleotide mit der allgemeinen Strukturformel (I) enthält: 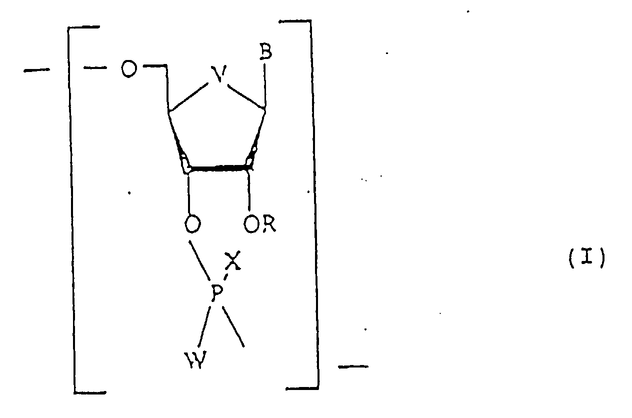
worin
B eine Nukleosidbase darstellt, die insbesondere aus der Gruppe, bestehend aus Adenin-9-yl (A), Cytosin-1-yl (C), Guanin-9-yl (G), Uracil-1-yl (U), Uracil-5-yl (ψ), Hypoxanthin-9-yl (I), Thymin-1-yl (T) und 2-Aminoadenin-9-yl ausgewählt ist,
V bei jedem Nukleotid unabhängig eine O- oder ein CH₂-Gruppe ist,
X und W jeweils in einem Nukleotid gleich oder verschieden sein können und unabhängig voneinander O-, S-, NH₂-, Alkyl- oder Alkoxygruppen mit 1 bis 10, vorzugsweise mit 1 bis 4 Kohlenstoffatomen sind,
R Wasserstoff oder eine geradkettige oder verzweigte, gegebenenfalls mit Halogen-, Cyano-, Isocyano-, Nitro-, Amino-, Carboxyl-, Hydroxyl- oder/und Mercaptogruppen substituierte Alkyl-, Alkenyl- oder Alkinylgruppe mit 1 bis 10 Kohlenstoffatomen ist,
und worin bei den Nukleotiden N₁, N₄, N₈ und N₁₁ in Formel (II) der Rest R in Formel (I) Wasserstoff ist und bei mindestens einem der Nukleotide N₂, N₃, N₅, N₆, N₇, N₉, N₁₀, N₁₂, N₁₃ und N₁₄ in Formel (II) der Rest R in Formel (I) von Wasserstoff verschieden ist.

2. Verfahren nach Anspruch 1,
**dadurch gekennzeichnet,**
daß die Reste B in Formel (I) bei den Nukleotiden N₁, N₄, N₈ bzw. N₁₁ jeweils A oder 2-Aminoadenin-9-yl, G,G bzw. G sind.

3. Verfahren nach einem der vorhergehenden Ansprüche,
**dadurch gekennzeichnet,**
daß die Reste V, W und X in Formel (I) O-Gruppen sind.

4. Verfahren nach einem der vorhergehenden Ansprüche,
**dadurch gekennzeichnet,**
daß die von Wasserstoff verschiedenen Reste R in Formel (I) 1 bis 6 Kohlenstoffatome enthalten.

5. Verfahren nach Anspruch 4,
**dadurch gekennzeichnet,**
daß die von Wasserstoff verschiedenen Reste R in Formel (I) aus der Gruppe, bestehend aus Methyl-, Ethyl-, Propyl, Allyl-, Dimethylallyl-, Butyl- oder Cyanomethylresten ausgewählt sind.

6. Verfahren nach einem der vorhergehenden Ansprüche,
**dadurch gekennzeichnet,**
daß die Oligonukleotidstruktur die allgemeine Strukturformel (III) aufweist: 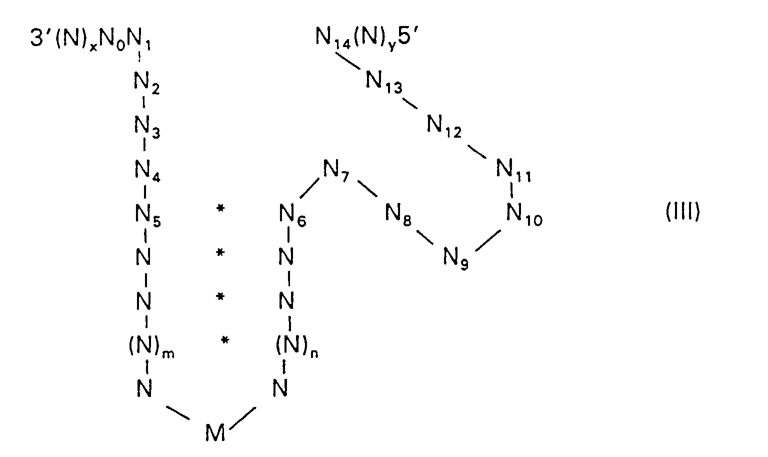 worin
N, x und y wie in Anspruch 1 definiert sind,
M eine chemische Bindung darstellt oder eine Nukleotidsequenz (N)ₐ bedeutet, wobei a ≥ 1 ist, m und n gleich oder verschieden sind und gegebenenfalls ein oder mehrere zusätzliche Nukleotide nach N₇ oder/und N₉ isertiert werden können.

7. Verfahren nach Anspruch 6,
**dadurch gekennzeichnet,**
daß die Reste R in Formel (III) bei den Nukleotiden N₁, N₄, N₈, N₁₀, N₁₁ und N₁₂ Wasserstoff sind.

8. Verfahren nach Anspruch 6 oder 7,
**dadurch gekennzeichnet,**
daß die Reste R in Formel (III) bei allen Nukleotiden außer N₁, N₄, N₈, N₁₀, N₁₁ und N₁₂ von Wasserstoff verschieden sind.

9. Verfahren nach einem der vorhergehenden Ansprüche,
**dadurch gekennzeichnet,**
daß die Reste V, W und X in Formel (I) O-Gruppen sind und daß bei den Nukleotiden N₁ bis N₁₄ in Formel (I) oder (III) die Reste B und R die folgenden Bedeutungen besitzen:
N₁ : B = A und R = H,
N₂ : B = A und R = Allyl,
N₃ : B = A und R = Allyl,
N₄ : B = G unf R = H,
N₅ : B = C und R = Allyl,
N₆ : B = G und R = Allyl,
N₇ : B = A und R = Allyl,
N₈ : B = G und R = H,
N₉ : B = U und R = Allyl,
N₁₀ : B = A und R = H,
N₁₁ : B = G und R = H,
N₁₂ : B = U und R = H,
N₁₃ : B = C und R = Allyl,
N₁₄ : B = U und R = Allyl.

10. Verfahren nach einem der Ansprüche 1 bis 8,
**dadurch gekennzeichnet,**
daß die Reste V, W und X in Formel (I) O-Gruppen sind, mit der Ausnahme, daß die Verbindung zwischen N₁₁ und N₁₂ eine Phosphorthioatgruppe (X = O und W = S) ist, und daß bei den Nukleotiden N₁ bis N₁₄ in Formel (I) oder (III) die Reste B und R die Bedeutung gemäß Anspruch 9 besitzen.

11. Verfahren nach einem der Ansprüche 1 bis 8,
**dadurch gekennzeichnet,**
daß die Reste V, W und X in Formel (I) O-Gruppen sind und daß bei den Nukleotiden N₁ bis N₁₄ in Formel (I) oder (III) die Reste B die Bedeutung gemäß Anspruch 9 besitzen und daß für N₁, N₄, N₈, N₁₀, N₁₁ und N₁₂ R = H, für N₉ R = 3,3-Dimethylallyl und für N₂, N₃, N₅, N₆, N₇, N₁₃ und N₁₄ R = Allyl ist.

12. Verfahren nach einem der Ansprüche 1 bis 8,
**dadurch gekennzeichnet,**
daß die Reste V, W und X in Formel (I) O-Gruppen sind und daß bei den Nukleotiden N₁ bis N₁₄ in Formel (I) oder (III) die Reste B die Bedeutung gemäß Anspruch 9 besitzen und daß für N₁, N₄, N₈, N₁₀, N₁₁ und N₁₂ R = H, für N₃ R = 3,3-Dimethylallyl und für N₂, N₅, N₆, N₇, N₉, N₁₃ und N₁₄ R = Allyl ist.

13. Verfahren nach einem der Ansprüche 1 bis 8,
**dadurch gekennzeichnet,**
daß die Reste V, W und X in Formel (I) O-Gruppen sind und daß bei den Nukleotiden N₁ bis N₁₄ in Formel (I) oder (III) die Reste B die Bedeutung gemäß Anspruch 9 besitzen und daß für N₁, N₄, N₈, N₁₀, N₁₁ und N₁₂ R = H, für N₂, N₃, N₇, N₉ und N₁₃ R = Cyanomethyl und für N₅, N₆ und N₁₄ R = Allyl ist.

14. Verfahren nach einem der Ansprüche 1 bis 8,
**dadurch gekennzeichnet,**
daß die Reste V, W und X in Formel (I) O-Gruppen sind und daß bei den Nukleotiden N₁ bis N₁₄ in Formel (I) oder (III) die Reste B die Bedeutung gemäß Anspruch 9 besitzen und daß für N₁, N₄, N₈, N₁₀, N₁₁ und N₁₂ R = H, für N₅ und N₆ R = Butyl und für N₂, N₃, N₇, N₉, N₁₃ und N₁₄ R = Allyl ist, wobei gegebenenfalls für einen oder mehrere der Reste N, die in Formel (III) in basengepaarter Form vorliegen, R = Butyl ist.

15. Verfahren nach einem der vorhergehenden Ansprüche,
**dadurch gekennzeichnet,**
daß sich an den freien 3'-Enden der Oligonukleotidstruktur 3'-Deoxyribonukleotide oder/und 3'-O-Alkylribonukleotide befinden.

16. Verfahren nach einem der vorhergehenden Ansprüche,
**dadurch gekennzeichnet,**
daß die Oligonukleotidstruktur weiterhin zur Stabilisierung ihrer räumlichen Konfiguration Nukleotide enthält, deren Nukleosidbasen durch ein Quervernetzungsmittel modifiziert sind.

17. Verfahren nach Anspruch 16,
**dadurch gekennzeichnet,**
daß das Quervernetzungsmittel Psoralen oder ein Psoralenderivat ist.

18. Verfahren nach einem der vorhergehenden Ansprüche,
**dadurch gekennzeichnet,**
daß die Oligonukleotidstruktur mit einer prosthetischen Gruppe, ausgewählt aus Polyaminosäuren, Lipiden, Hormonen oder Peptiden verknüpft wird, um ihr die Aufnahme in der Zelle oder/und die spezifische zelluläre Lokalisierung zu verbessern.

19. Verfahren zur Spaltung einer Nukleinsäure-Zielsequenz unter Verwendung einer synthetischen katalytischen Oligonukleotidstruktur hergestellt durch das Verfahren nach einem der Ansprüche 1 bis 18.

20. Verfahren nach Anspruch 19,
**dadurch gekennzeichnet,**
daß die Nukleinsäure-Zielsequenz ein von der synthetischen katalytischen Oligonukleotidstruktur verschiedenes Molekül ist.

21. Verfahren nach einem der Ansprüche 19 oder 20,
**dadurch gekennzeichnet,**
daß man eine Nukleinsäure-Zielsequenz der allgemeinen Formel (IV) unter Verwendung eines Oligonukleotids mit der allgemeinen Formel (II) spaltet, wobei eine Zwischenstufe mit der folgenden Struktur ausgebildet wird, 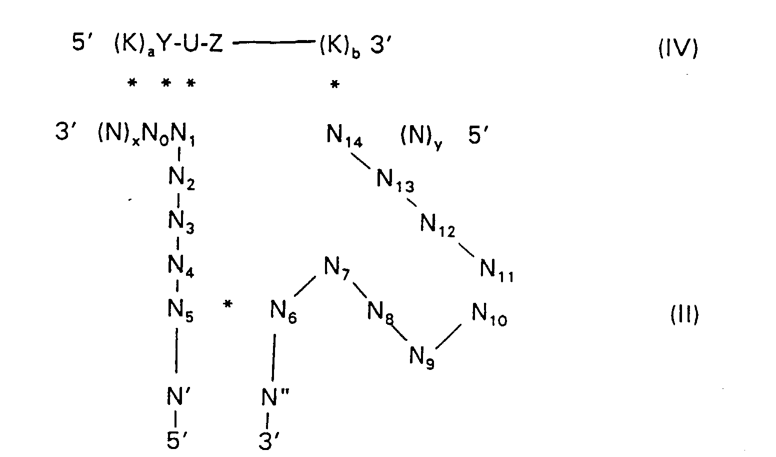 wobei
die Symbole N, N', N", x, y und * wie in Anspruch 1 definiert sind,
und K, Y, U und Z Nukleotide der Nukleinsäure-Zielsequenz darstellen, worin U Uridin ist,
Z ein nicht modifiziertes Ribonukleotid, ausgewählt aus der Gruppe, bestehend aus Adenosin, Cytidin oder Uridin ist,
K und Y beliebige Nukleotide sind,
a ≥ 1 und b ≥ 3 ist,
und die Spaltung der Nukleinsäure-Zielsequenz 3'-seitig der Nukleotidsequenz YUZ erfolgt, und wobei gegebenenfalls eine chemische Bindung zwischen dem 5'-Ende der Nukleinsäure-Zielsequenz (IV) und dem 3'-Ende des Oligonukleotids (II) bei (N)ₓ oder zwischen dem 3'-Ende der Nukleinsäure-Zielsequenz (IV) und dem 5'-Ende des Oligonukleotids (II) bei (N)_{y} existiert.

22. Verfahren nach Anspruch 21,
**dadurch gekennzeichnet,**
daß Y Guanosin ist.

23. Verfahren nach Anspruch 21 oder 22,
**dadurch gekennzeichnet,**
daß Z Adenosin oder Cytidin ist.

24. Verfahren nach einem der Ansprüche 19 bis 23,
**dadurch gekennzeichnet,**
daß die Nukleinsäure-Zielsequenz eine Ribonukleinsäure ist.

25. Verfahren nach einem der Ansprüche 19 bis 24,
**dadurch gekennzeichnet,**
daß man die Spaltung in Gegenwart eines divalenten Kations, insbesondere Mg²⁺ durchführt.

26. Verfahren nach einem der Ansprüche 19 bis 24,
**dadurch gekennzeichnet,**
daß man die Spaltung bei einem pH-Wert von etwa 7 bis 9 durchführt.

27. Verwendung einer Oligonukleotidstruktur, die durch ein Verfahren nach einem der Ansprüche 1 bis 18 hergestellt wurde, zur katalytischen Spaltung einer Nukleinsäure-Zielsequenz.

28. Verfahren zur Herstellung eines Arzneimittels,
**dadurch gekennzeichnet,**
daß man als Wirkstoff eine Oligonukleotidstruktur, die durch ein Verfahren nach einem der Ansprüche 1 bis 18 hergestellt ist, verwendet und gegebenenfalls zusammen mit üblichen pharmazeutischen Träger-, Hilfs-, Füll- oder/und Verdünnungsmitteln formuliert.

29. Verfahren zur Herstellung eines Arzneimittels für die antivirale Therapie in Menschen, Tieren und Pflanzen,
**dadurch gekennzeichnet,**
daß man als Wirkstoff eine Oligonukleotidstruktur, die durch ein Verfahren nach einem der Ansprüche 1 bis 18 hergestellt ist, verwendet.

30. Diagnostisches Reagenz,
**dadurch gekennzeichnet,**
daß es als Bestandteil eine Oligonukleotidstruktur enthält, die durch ein Verfahren nach einem der Ansprüche 1 bis 18 hergestellt ist.

31. Verfahren zur Herstellung eines diagnostischen Reagenz für eine genetische Musterungsprozedur,
**dadurch gekennzeichnet,**
daß es als Bestandteil eine Oligonukleotidstruktur enthält, die durch ein Verfahren nach einem der Ansprüche 1 bis 18 hergestellt ist.

## Claims (Claims for the following Contracting State(s): AT, BE, CH, LI, DE, FR, GB, IT, LU, MC, NL, PT, SE)

1. Synthetic catalytic oligonucleotide structure which is suitable for hybridizing to a nucleic acid target sequence and for cleaving a nucleic acid target sequence and has an oligonucleotide structure of the general structural formula (II) 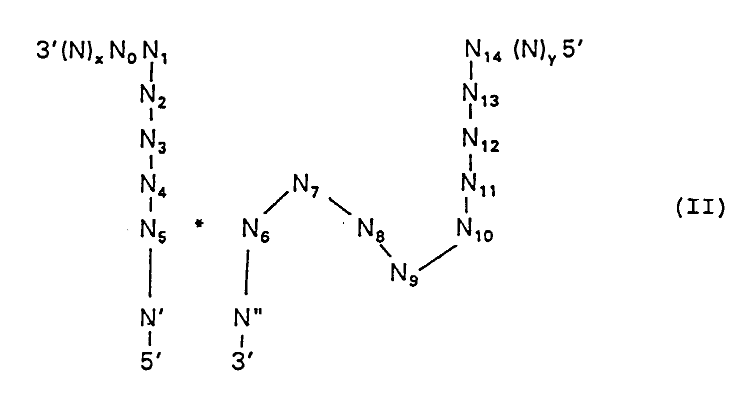 in which
N in each case represents a nucleotide according to the general structural formula (I),
x and y can be the same or different and x ≥ 1 and y ≥ 2,
N₅ and N₆ are in each case nucleotides which are complementary to one another and * represents a base pairing,
N' and N" either represent two nucleotide sequences which contain nucleotides which are at least partially complementary to one another thus enabling a stable base pairing between the two nucleotide sequences
or
N' and N" together represent a single nucleotide sequence whereby at least part of the sequence can form a double-stranded stem by base pairing between complementary nucleotides,
and wherein one or several additional nucleotides N can optionally be inserted after N₇ and/or N₉,
and wherein the oligonucleotide structure contains nucleotides of the general structural formula (I): 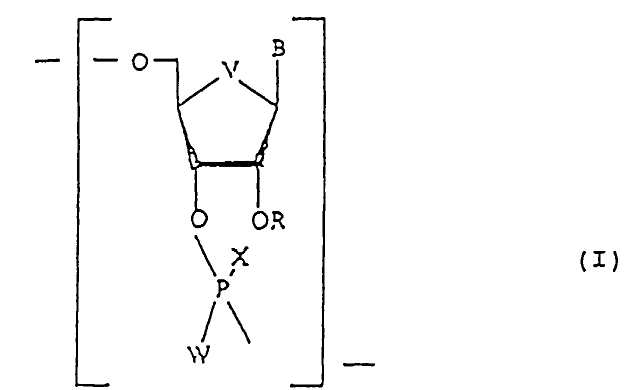
in which
B represents a nucleoside base which is in particular selected from the group comprising adenin-9-yl (A), cytosin-1-yl (C), guanin-9-yl (G), uracil-1-yl (U), uracil-5-yl (y), hypoxanthin-9-yl (I), thymin-1-yl (T) and 2-aminoadenin-9-yl,
V in each nucleotide is independently an O or a CH₂ group,
X and W can in each nucleotide be the same or different and are independently of one another O, S, NH₂, alkyl or alkoxy groups with 1 to 10, preferably with 1 to 4 carbon atoms,
R is hydrogen or a straight-chained or branched alkyl, alkenyl or alkinyl group with 1 to 10 carbon atoms which is optionally substituted by halogen, cyano, isocyano, nitro, amino, carboxyl, hydroxyl or/and mercapto groups
and wherein in the nucleotides N₁, N₄, N₈ and N₁₁ in formula (II) the residue R in formula (I) is hydrogen and in at least one of the nucleotides N₂, N₃, N₅, N₆, N₇, N₉, N₁₀, N₁₂, N₁₃ and N₁₄ in formula (II) the residue R in formula (I) is different from hydrogen.

2. Oligonucleotide structure as claimed in claim 1,
**wherein**
the residues B in the formula (I) in the nucleotides N₁, N₄, N₈ or N₁₁ are in each case A or 2-aminoadenin-9-yl, G, G and G respectively.

3. Oligonucleotide structure as claimed in one of the previous claims,
**wherein**
the residues V, W and X in formula (I) are O groups.

4. Oligonucleotide structure as claimed in one of the previous claims,
**wherein**
the residues R in formula (I) which are different from hydrogen contain 1 to 6 carbon atoms.

5. Oligonucleotide structure as claimed in claim 4,
**wherein**
the residues R in formula (I) which are different from hydrogen are selected from the group comprising methyl, ethyl, propyl, allyl, dimethylallyl, butyl or cyanomethyl residues.

6. Oligonucleotide structure as claimed in one of the previous claims having the general structural formula (III): 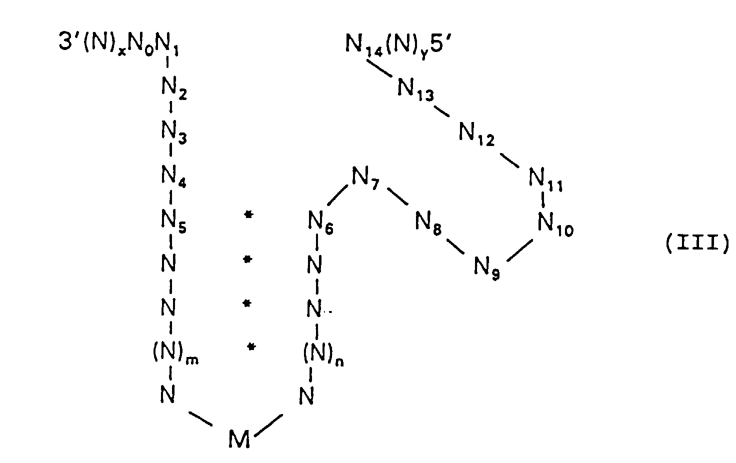 in which N, x and y are defined as in claim 1, M represents a chemical bond or denotes a nucleotide sequence (N)ₐ, in which a ≥ 1, m and n are the same or different and optionally one or several additional nucleotides can be inserted after N₇ or/and N₉.

7. Oligonucleotide structure as claimed in claim 6
**wherein**
the residues R in formula (III) are hydrogen in the nucleotides N₁, N₄, N₈, N₁₀, N₁₁ and N₁₂.

8. Oligonucleotide structure as claimed in claim 6 or 7,
**wherein**
the residues R in formula (III) are different from hydrogen in all nucleotides apart from N₁, N₄, N₈, N₁₀, N₁₁ and N₁₂.

9. Oligonucleotide structure as claimed in one of the previous claims,
**wherein**
the residues V, W and X in formula (I) are O groups and in formula (I) or (III) the residues B and R have the following meanings in the nucleotides N₁ to N₁₄:
N₁: B = A and R = H,
N₂: B = A and R = allyl,
N₃: B = A and R = allyl,
N₄: B = G and R = H,
N₅: B = C and R = allyl,
N₆: B = G and R = allyl,
N₇: B = A and R = allyl,
N₈: B = G and R = H,
N₉: B = U and R = allyl,
N₁₀: B = A and R = H,
N₁₁: B = G and R = H,
N₁₂: B = U and R = H,
N₁₃: B = C and R = allyl,
N₁₄: B = U and R = allyl.

10. Oligonucleotide structure as claimed in one of the claims 1 - 8,
**wherein**
the residues V, W and X in formula (I) are O groups with the exception that the linkage between N₁₁ and N₁₂ is a phosphorothioate group (X = O and W = S) and the residues B and R in formula (I) or (III) have the meaning according to claim 9 in the nucleotides N₁ to N₁₄.

11. Oligonucleotide structure as claimed in one of the claims 1 - 8,
**wherein**
the residues V, W and X in formula (I) are O groups and the residues B in formula (I) or (III) have the meaning according to claim 9 for the nucleotides N₁ to N₁₄ and R = H for N₁, N₄, N₈, N₁₀, N₁₁ and N₁₂, R = 3,3-dimethylallyl for N₉ and R = allyl for N₂, N₃, N₅, N₆, N₇, N₁₃ and N₁₄.

12. Oligonucleotide structure as claimed in one of the claims 1 - 8,
**wherein**
the residues V, W and X in formula (I) are O groups and the residues B in formula (I) or (III) have the meaning according to claim 9 for the nucleotides N₁ to N₁₄ and R = H for N₁, N₄, N₈, N₁₀, N₁₁ and N₁₂, R = 3,3-dimethylallyl for N₃ and R = allyl for N₂, N₅, N₆, N₇, N₉, N₁₃ and N₁₄.

13. Oligonucleotide structure as claimed in one of the claims 1 - 8,
**wherein**
the residues V, W and X in formula (I) are O groups and the residues B in formula (I) or (III) have the meaning according to claim 9 for the nucleotides N₁ to N₁₄ and R = H for N₁, N₄, N₈, N₁₀, N₁₁ and N₁₂, R = cyanomethyl for N₂, N₃, N₇, N₉ and N₁₃ and R = allyl for N₅, N₆ and N₁₄.

14. Oligonucleotide structure as claimed in one of the claims 1 - 8,
**wherein**
the residues V, W and X in formula (I) are O groups and the residues B in formula (I) or (III) have the meaning according to claim 9 for the nucleotides N₁ to N₁₄ and R = H for N₁, N₄, N₈, N₁₀, N₁₁ and N₁₂, R = butyl for N₅ and N₆ and R = allyl for N₂, N₃, N₇, N₉, N₁₃ and N₁₄ and optionally R = butyl in one or several of the residues N that are present in a base-paired form in formula (III).

15. Oligonucleotide structure as claimed in one of the previous claims,
**wherein**
3'-deoxyribonucleotides or/and 3'-O-alkylribonucleotides are present at the free 3'-ends of the oligonucleotide structure.

16. Oligonucleotide structure as claimed in one of the previous claims,
**wherein**
it additionally contains nucleotides to stabilize its spatial configuration whose nucleoside bases are modified by a cross-linking agent.

17. Oligonucleotide structure as claimed in claim 16,
**wherein**
the cross-linking agent is psoralen or a psoralen derivative.

18. Oligonucleotide structure as claimed in one of the previous claims,
**wherein**
it is linked to a prosthetic group selected from polyamino acids, lipids, hormones or peptides in order to improve the uptake into the cell or/and the specific cellular localization of the oligonucleotide structure.

19. Process for cleaving a nucleic acid target sequence using a synthetic catalytic oligonucleotide structure as claimed in one of the claims 1 to 18.

20. Process as claimed in claim 19,
**wherein**
the nucleic acid target sequence is a molecule which is different from the synthetic catalytic oligonucleotide structure.

21. Process as claimed in one of the claims 19 or 20,
**wherein**
a nucleic acid target sequence of the general formula (IV) is dleaved using an oligonucleotide of the general formula (II) in the process of which an intermediate stage forms having the following structure: 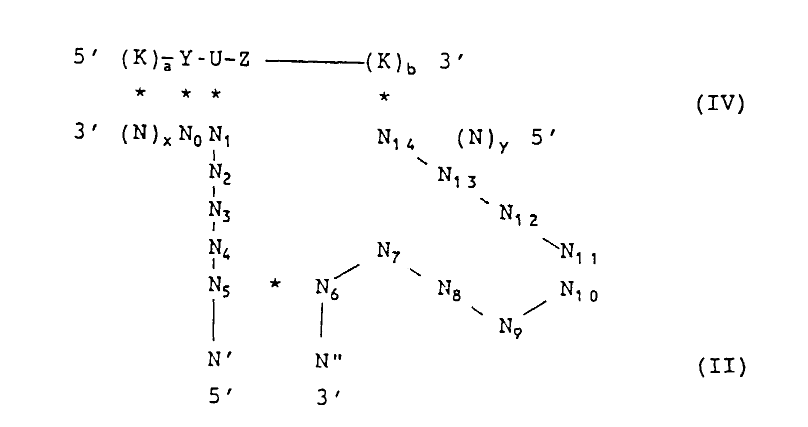 in which
the symbols N, N', N", x, y and * are defined as in claim 1,
and K, Y, U and Z represent nucleotides of the nucleic acid target sequence
in which U is uridine,
Z is a non-modified ribonucleotide selected from the group comprising adenosine, cytidine or uridine,
K and Y are any nucleotides,
a ≥ 1 and b ≥ 3,
and the cleavage of the nucleic acid target sequence takes place on the 3'-side of the nucleotide sequence YUZ and there is optionally a chemical bond between the 5'-end of the nucleic acid target sequence (IV) and the 3'-end of the oligonucleotide (II) at (N)ₓ or between the 3'-end of the nucleic acid target sequence (IV) and the 5'-end of the oligonucleotide (II) at (N)_{y}.

22. Process as claimed in claim 21,
**wherein**
Y is guanosine.

23. Process as claimed in claim 21 or 22,
**wherein**
Z is adenosine or cytidine.

24. Process as claimed in one of the claims 19 to 23,
**wherein**
the nucleic acid target sequence is a ribonucleic acid.

25. Process as claimed in one of the claims 19 to 24,
**wherein**
the cleavage is carried out in the presence of a divalent cation, in particular Mg²⁺.

26. Process as claimed in one of the claims 19 to 24,
**wherein**
the cleavage is carried out at a pH value of about 7 to 9.

27. Use of an oligonucleotide structure as claimed in one of the claims 1 to 18 for the catalytic cleavage of a nucleic acid target sequence.

28. Pharmaceutical preparation,
**wherein**
it contains an oligonucleotide structure as claimed in one of the claims 1 to 18 as the active substance optionally together with common pharmaceutical carriers, auxiliary substances, fillers or/and diluents.

29. Process for the production of a pharmaceutical preparation for antiviral therapy in humans, animals and plants,
**wherein**
an oligonucleotide structure as claimed in one of the claims 1 to 18 is used as the active substance.

30. Diagnostic reagent,
**wherein**
it contains an oligonucleotide structure as claimed in one of the claims 1 to 18 as a constituent.

31. Process for the production of a diagnostic reagent for a genetic screening procedure,
**wherein**
it contains an oligonucleotide structure as claimed in one of the claims 1 to 18 as a constituent.

## Claims (Claims for the following Contracting State(s): ES)

1. Process for the production of synthetic catalytic oligonucleotide structures composed of monomer units wherein the oligonucleotide structure is suitable for hybridizing to and for cleaving a nucleic acid target sequence and has an oligonucleotide structure of the general structural formula (II) 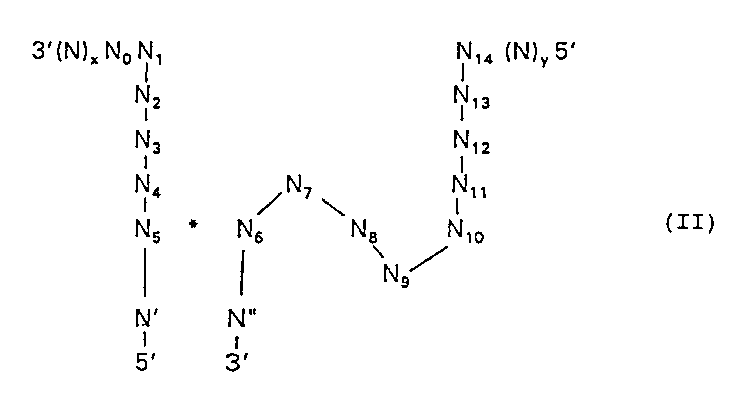 in which
N in each case represents a nucleotide according to the general structural formula (I), x and y can be the same or different and x ≥ 1 and y ≥ 2,
N₅ and N₆ are in each case nucleotides which are complementary to one another and * represents a base pairing,
N' and N" either represent two nucleotide sequences which contain nucleotides which are at least partially complementary to one another so as to enable a stable base pairing between the two nucleotide sequences
or
N' and N" together represent a single nucleotide sequence in which at least part of the sequence can form a double-stranded stem by base pairing between complementary nucleotides,
and in which one or several additional nucleotides N can optionally be inserted after N₇ and/or N₉,
and in which the oligonucleotide structure contains nucleotides of the general structural formula (I): 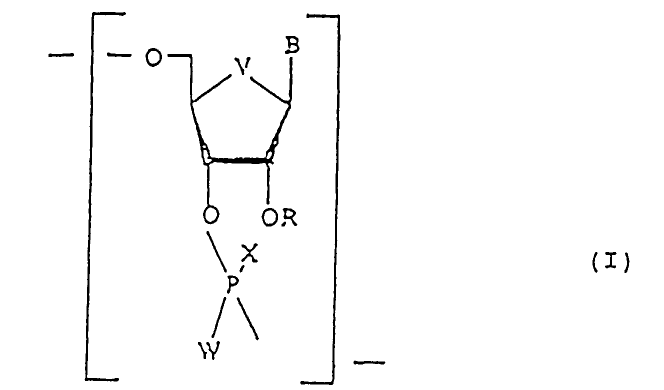
in which
B represents a nucleoside base which is in particular selected from the group comprising adenin-9-yl (A), cytosin-1-yl (C), guanin-9-yl (G), uracil-1-yl (U), uracil-5-yl (y), hypoxanthin-9-yl (I), thymin-1-yl (T) and 2-aminoadenin-9-yl,
V in each nucleotide is independently an O or a CH₂ group,
X and W can in each nucleotide be the same or different and are independently of one another O, S, NH₂, alkyl or alkoxy groups with 1 to 10, preferably with 1 to 4 carbon atoms,
R is hydrogen or a straight-chained or branched alkyl, alkenyl or alkinyl group with 1 to 10 carbon atoms which is optionally substituted by halogen, cyano, isocyano, nitro, amino, carboxyl, hydroxyl or/and mercapto groups
and wherein in the nucleotides N₁, N₄, N₈ and N₁₁ in formula (II) the residue R in formula (I) is hydrogen and in at least one of the nucleotides N₂, N₃, N₅, N₆, N₇, N₉, N₁₀, N₁₂, N₁₃ and N₁₄ in formula (II) the residue R in formula (I) is different from hydrogen.

2. Process as claimed in claim 1,
**wherein**
the residues B in the formula (I) in the nucleotides N₁, N₄, N₈ or N₁₁ are in each case A or 2-aminoadenin-9-yl, G, G and G respectively.

3. Process as claimed in one of the previous claims,
**wherein**
the residues V, W and X in formula (I) are O groups.

4. Process as claimed in one of the previous claims,
**wherein**
the residues R in formula (I) which are different from hydrogen contain 1 to 6 carbon atoms.

5. Process as claimed in claim 4,
**wherein**
the residues R in formula (I) which are different from hydrogen are selected from the group comprising methyl, ethyl, propyl, allyl, dimethylallyl, butyl or cyanomethyl residues.

6. Process as claimed in one of the previous claims wherein the oligonucleotide structure has the general structural formula (III): 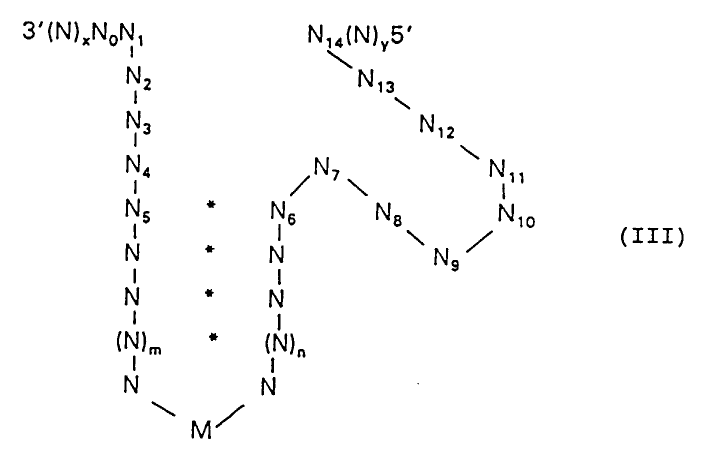 in which N, x and y are defined as in claim 1, M represents a chemical bond or denotes a nucleotide sequence (N)ₐ, in which a ≥ 1, m and n are the same or different and optionally one or several additional nucleotides can be inserted after N₇ or/and N₉.

7. Process as claimed in claim 6
**wherein**
the residues R in formula (III) are hydrogen in the nucleotides N₁, N₄, N₈, N₁₀, N₁₁ and N₁₂.

8. Process as claimed in claim 6 or 7,
**wherein**
the residues R in formula (III) are different from hydrogen in all nucleotides apart from N₁, N₄, N₈, N₁₀, N₁₁ and N₁₂.

9. Process as claimed in one of the previous claims,
**wherein**
the residues V, W and X in formula (I) are O groups and the residues B and R in formula (I) or (III) have the following meanings for the nucleotides N₁ to N₁₄:
N₁: B = A and R = H,
N₂: B = A and R = allyl,
N₃: B = A and R = allyl,
N₄: B = G and R = H,
N₅: B = C and R = allyl,
N₆: B = G and R = allyl,
N₇: B = A and R = allyl,
N₈: B = G and R = H,
N₉: B = U and R = allyl,
N₁₀: B = A and R = H,
N₁₁: B = G and R = H,
N₁₂: B = U and R = H,
N₁₃: B = C and R = allyl,
N₁₄: B = U and R = allyl.

10. Process as claimed in one of the claims 1 to 8,
**wherein**
the residues V, W and X in formula (I) are O groups with the exception that the linkage between N₁₁ and N₁₂ is a phosphorothioate group (X = O and W = S) and the residues B and R in formula (I) or (III) have the meaning according to claim 9 for the nucleotides N₁ to N₁₄.

11. Process as claimed in one of the claims 1 to 8,
**wherein**
the residues V, W and X in formula (I) are O groups and the residues B in formula (I) or (III) have the meaning according to claim 9 for the nucleotides N₁ to N₁₄ and R = H for N₁, N₄, N₈, N₁₀, N₁₁ and N₁₂, R = 3,3-dimethylallyl for N₉ and R = allyl for N₂, N₃, N₅, N₆, N₇, N₁₃ and N₁₄.

12. Process as claimed in one of the claims 1 to 8,
**wherein**
the residues V, W and X in formula (I) are O groups and the residues B in formula (I) or (III) have the meaning according to claim 9 for the nucleotides N₁ to N₁₄ and R = H for N₁, N₄, N₈, N₁₀, N₁₁ and N₁₂, R = 3,3-dimethylallyl for N₃ and R = allyl for N₂, N₅, N₆, N₇, N₉, N₁₃ and N₁₄.

13. Process as claimed in one of the claims 1 to 8,
**wherein**
the residues V, W and X in formula (I) are O groups and the residues B in formula (I) or (III) have the meaning according to claim 9 for the nucleotides N₁ to N₁₄ and R = H for N₁, N₄, N₈, N₁₀, N₁₁ and N₁₂, R = cyanomethyl for N₂, N₃, N₇, N₉ and N₁₃ and R = allyl for N₅, N₆ and N₁₄.

14. Process as claimed in one of the claims 1 to 8,
**wherein**
the residues V, W and X in formula (I) are O groups and the residues B in formula (I) or (III) have the meaning according to claim 9 for the nucleotides N₁ to N₁₄ and R = H for N₁, N₄, N₈, N₁₀, N₁₁ and N₁₂, R = butyl for N₅ and N₆ and R = allyl for N₂, N₃, N₇, N₉, N₁₃ and N₁₄ and optionally R = butyl in one or several of the residues N that are present in a base-paired form in formula (III).

15. Process as claimed in one of the previous claims,
**wherein**
3'-deoxyribonucleotides or/and 3'-O-alkylribonucleotides are present at the free 3'-ends of the oligonucleotide structure.

16. Process as claimed in one of the previous claims,
**wherein**
it additionally contains nucleotides to stabilize its spatial configuration whose nucleoside bases are modified by a cross-linking agent.

17. Process as claimed in claim 16,
**wherein**
the cross-linking agent is psoralen or a psoralen derivative.

18. Process as claimed in one of the previous claims,
**wherein**
the oligonucleotide structure is linked to a prosthetic group selected from polyamino acids, lipids, hormones or peptides in order to improve the uptake into the cell or/and the specific cellular localization.

19. Process for cleaving a nucleic acid target sequence using a synthetic catalytic oligonucleotide structure as claimed in one of the claims 1 to 18.

20. Process as claimed in claim 19,
**wherein**
the nucleic acid target sequence is a molecule which is different from the synthetic catalytic oligonucleotide structure.

21. Process as claimed in one of the claims 19 or 20,
**wherein**
a nucleic acid target sequence of the general formula (IV) is cleaved using an oligonucleotide of the general formula (II) in the process of which an intermediate stage forms having the following structure: 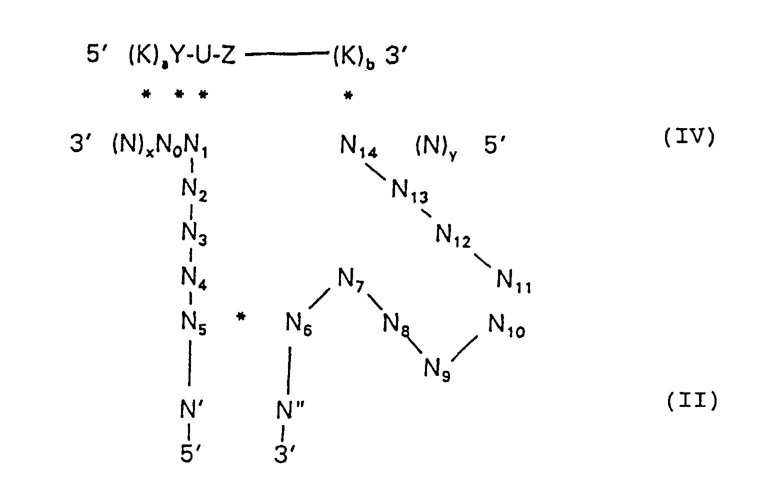 in which
the symbols N, N', N", x, y and * are defined as in claim 1,
and K, Y, U and Z represent nucleotides of the nucleic acid target sequence in which U is uridine,
Z is a non-modified ribonucleotide selected from the group comprising adenosine, cytidine or uridine,
K and Y are any nucleotides,
a ≥ 1 and b ≥ 3,
and the cleavage of the nucleic acid target sequence takes place on the 3'-side of the nucleotide sequence YUZ and in which there is optionally a chemical bond between the 5'-end of the nucleic acid target sequence (IV) and the 3'-end of the oligonucleotide (II) at (N)ₓ or between the 3'-end of the nucleic acid target sequence (IV) and the 5'-end of the oligonucleotide (II) at (N)y.

22. Process as claimed in claim 21,
**wherein**
Y is guanosine.

23. Process as claimed in claim 21 or 22,
**wherein**
Z is adenosine or cytidine.

24. Process as claimed in one of the claims 19 to 23,
**wherein**
the nucleic acid target sequence is a ribonucleic acid.

25. Process as claimed in one of the claims 19 to 24,
**wherein**
the cleavage is carried out in the presence of a divalent cation, in particular Mg²⁺.

26. Process as claimed in one of the claims 19 to 24,
**wherein**
the cleavage is carried out at a pH value of about 7 to 9.

27. Use of an oligonucleotide structure which has been prepared by a process as claimed in one of the claims 1 to 18 for the catalytic cleavage of a nucleic acid target sequence.

28. Process for the production of a pharmaceutical preparation,
**wherein**
an oligonucleotide structure prepared by a process as claimed in one of the claims 1 to 18 is used as the active substance and is optionally formulated together with common pharmaceutical carriers, auxiliary substances, fillers or/and diluents.

29. Process for the production of a pharmaceutical preparation for antiviral therapy in humans, animals and plants,
**wherein**
an oligonucleotide structure which is prepared by a process as claimed in one of the claims 1 to 18 is used as the active substance.

30. Diagnostic reagent,
**wherein**
it contains an oligonucleotide structure as a constituent which is prepared by a process as claimed in one of the claims 1 to 18.

31. Process for the production of a diagnostic reagent for a genetic screening procedure,
**wherein**
it contains an oligonucleotide structure as a constituent which is prepared by a process as claimed in one of the claims 1 to 18 .

## Revendications (Revendications pour l'(les) Etat(s) contractant(s) suivant(s): AT, BE, CH, LI, DE, FR, GB, IT, LU, MC, NL, PT, SE)

1. Structure oligonucléotidique catalytique synthétique, appropriée à l'hybridation avec une séquence d'acides nucléiques cible et à sa coupure, et qui a une structure oligonucléotidique ayant la formule structurale générale (II): 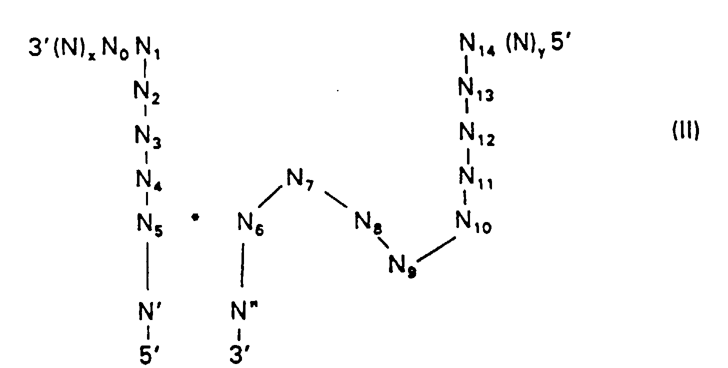 dans laquelle
les N représentent chacun un nucléotide de formule structurale générale (I),
x et y peuvent être identiques ou différents et x est ≥ 1 et y ≥ 2,
N₅ et N₆ sont des nucléotides complémentaires l'un de l'autre et * représente un appariement de bases,
N' et N" représentent deux séquences de nucléotides contenant des nucléotides au moins partiellement complémentaires les uns des autres, de façon à permettre un appariement de bases stable entre les deux séquences de nucléotides,
ou bien
N' et N" représentent ensemble une seule séquence de nucléotides, au moins une partie de la séquence pouvant former un tronc double brin par appartement de bases entre des nucléotides complémentaires,
et un ou plusieurs nucléotides N supplémentaires peuvent éventuellement être insérés après N₇ et/ou N₉,
et la structure oligonucléotidique contient des nucléotides ayant la formule structurale générale (I): 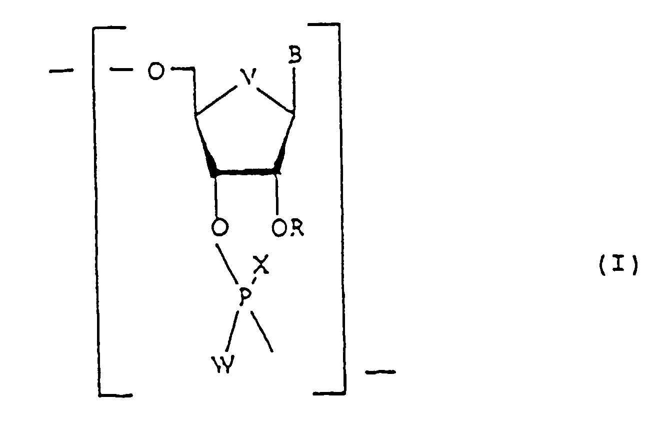
dans laquelle
B représente une base de nucléoside choisie en particulier dans le groupe constitué par les restes adénine-9-yle (A), cytosine-1-yle (C), guanine-9-yle (G), uracile-1-yle (U), uracile-5-yle (ψ), hypoxanthine-9-yle (I), thymine-1-yle (T) et 2-aminoadénine-9-yle,
V est indépendamment, dans chaque nucléotide, un atome d'oxygène ou un groupe CH₂,
X et W peuvent être identiques ou différents dans chaque nucléotide, et représentent indépendamment l'un de l'autre un atome d'oxygène ou de soufre ou un groupe NH₂, alkyle ou alcoxy de 1 à 10, de préférence 1 à 4 atomes de carbone,
R est un atome d'hydrogène ou un groupe alkyle, alcényle ou alcynyle de 1 à 10 atomes de carbone linéaire ou ramifié, éventuellement substitué par des restes halogéno, cyano, isocyano, nitro, amino, carboxyle, hydroxyle et/ou mercapto,
et, dans les nucléotides N₁, N₄, N₈ et N₁₁ de la formule (II), le reste R de la formule (I) est un atome d'hydrogène, et, dans au moins l'un des nucléotides N₂, N₃, N₅, N₆, N₇, N₉, N₁₀, N₁₂, N₁₃ et N₁₄ de la formule (II), le reste R de la formule (I) est différent d'un atome d'hydrogène.

2. Structure oligonucléotidique selon la revendication 1, caractérisée en ce que, dans les nucléotides N₁, N₄, N₈ et N₁₁, les restes B de la formule (I) sont respectivement A ou 2-aminoadénine-9-yle, G, G et G.

3. Structure oligonucléotidique selon l'une des revendications précédentes, caractérisée en ce que les restes V, W et X de la formule (I) sont des atomes d'oxygène.

4. Structure oligonucléotidique selon l'une des revendications précédentes, caractérisée en ce que, dans la formule (I), les restes R différents de l'hydrogène contiennent de 1 à 6 atomes de carbone.

5. Structure oligonucléotidique selon la revendication 4, caractérisée en ce que, dans la formule (I), les restes R différents de l'hydrogène sont choisis dans le groupe constitué par les restes méthyle, éthyle, propyle, allyle, diméthylallyle, butyle ou cyanométhyle.

6. Structure oligonucléotidique selon l'une des revendications précédentes, ayant la formule structurale générale (III): 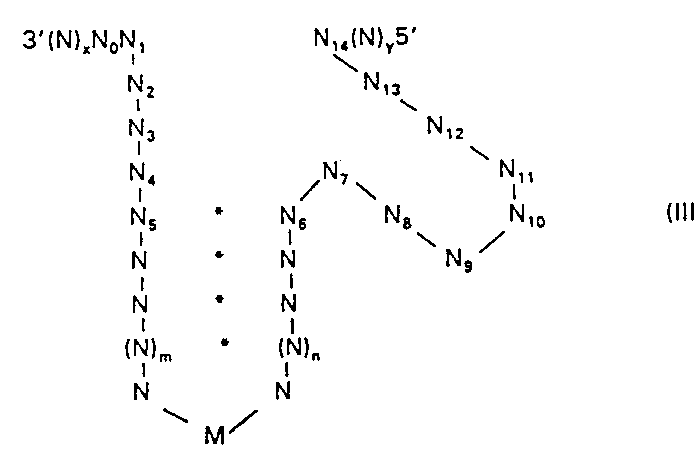 dans laquelle
N, x et y ont la définition donnée dans la revendication 1,
M représente une liaison chimique ou une séquence de nucléotides (N)ₐ dans laquelle a est ≥ 1, m et n sont identiques ou différents et un ou plusieurs nucléotides supplémentaires peuvent éventuellement être insérés après N₇ et/ou N₉.

7. Structure oligonucléotidique selon la revendication 6, caractérisée en ce que, dans la formule (III), les restes R représentent l'hydrogène dans les nucléotides N₁, N₄, N₈, N₁₀, N₁₁, et N₁₂.

8. Structure oligonucléotidique selon la revendication 6 ou 7, caractérisée en ce que, dans la formule (III), les restes R sont différents de l'hydrogène dans tous les nucléotides à l'exception de N₁, N₄, N₈, N₁₀, N₁₁ et N₁₂.

9. Structure oligonucléotidique selon l'une des revendications précédentes, caractérisée en ce que, dans la formule (I), les restes V, W et X sont des atomes d'oxygène et en ce que, dans les nucléotides N₁ à N₁₄ de la formule (I) ou (III), les restes B et R ont les significations suivantes:
N₁ : B = A et R = H,
N₂ : B = A et R = allyle,
N₃ : B = A et R = allyle,
N₄ : B = G et R = H,
N₅ : B = C et R = allyle,
N₆ : B = G et R = allyle,
N₇ : B = A et R = allyle,
N₈ : B = G et R = H,
N₉ : B = U et R = allyle,
N₁₀ : B = A et R = H,
N₁₁ : B = G et R = H,
N₁₂ : B = U et R = H,
N₁₃ : B = C et R = allyle,
N₁₄ : B = U et R = allyle.

10. Structure oligonucléotidique selon l'une des revendications 1 à 8, caractérisé en ce que, dans la formule (I), les restes V, W et X sont des atomes d'oxygène, avec l'exception selon laquelle la liaison entre N₁₁ et N₁₂ est un groupe phosphorothioate (X = O et W = S), et en ce que, dans les nucléotides N₁ à N₁₄ de la formule (I) ou (III), les restes B et R ont la signification donnée dans la revendication 9.

11. Structure oligonucléotidique selon l'une des revendications 1 à 8, caractérisé en ce que, dans la formule (I), les restes V, W et X sont des atomes d'oxygène, et en ce que, dans les nucléotides N₁ à N₁₄ de la formule (I) ou (III), les restes B ont la signification donnée dans la revendication 9, et en ce que, pour N₁, N₄, N₈, N₁₀, N₁₁ et N₁₂, R = H; pour N₉, R = 3,3-diméthylallyle et, pour N₂, N₃, N₅, N₆, N₇, N₁₃ et N₁₄, R = allyle.

12. Structure oligonucléotidique selon l'une des revendications 1 à 8, caractérisé en ce que, dans la formule (I), les restes V, W et X sont des atomes d'oxygène, et en ce que, dans les nucléotides N₁ à N₁₄ de la formule (I) ou (III), les restes B ont la signification donnée dans la revendication 9, et en ce que, pour N₁, N₄, N₈, N₁₀, N₁₁ et N₁₂, R = H; pour N₃, R = 3,3-diméthylallyle et, pour N₂, N₅, N₆, N₇, N₉, N₁₃ et N₁₄, R = allyle.

13. Structure oligonucléotidique selon l'une des revendications 1 à 8, caractérisé en ce que, dans la formule (I), les restes V, W et X sont des atomes d'oxygène, et en ce que, dans les nucléotides N₁ à N₁₄ de la formule (I) ou (III), les restes B ont la signification donnée dans la revendication 9, et en ce que, pour N₁, N₄, N₈, N₁₀, N₁₁ et N₁₂, R = H; pour N₂, N₃, N₇, N₉ et N₁₃, R = cyanométhyle et, pour N₅, N₆ et N₁₄, R = allyle.

14. Structure oligonucléotidique selon l'une des revendications 1 à 8, caractérisé en ce que, dans la formule (I), les restes V, W et X sont des atomes d'oxygène, et en ce que, dans les nucléotides N₁ à N₁₄ de la formule (I) ou (III), les restes B ont la signification donnée dans la revendication 9, et en ce que, pour N₁, N₄, N₈, N₁₀, N₁₁ et N₁₂, R=H; pour N₅ et N₆, R = butyle et, pour N₂, N₃, N₇, N₉, N₁₃ et N₁₄, R = allyle; R étant éventuellement butyle pour un ou plusieurs des restes N qui se trouvent sous une forme à bases appariées dans la formule (III).

15. Structure oligonucléotidique selon l'une des revendications précédentes, caractérisée en ce que des 3'-désoxyribonucléotides et/ou des 3'-O-alkylribonucléotides se trouvent aux extrémités 3' libres de la structure oligonucléotidique.

16. Structure oligonucléotidique selon l'une des revendications précédentes, caractérisée en ce qu'elle contient en outre, pour la stabilisation de sa configuration dans l'espace, des nucléotides dont les bases de nucléoside sont modifiées par un agent de réticulation transversale.

17. Structure oligonucléotidique selon la revendication 16, caractérisée en ce que l'agent de réticulation transversale est le psoralène ou un dérivé de psoralène.

18. Structure oligonucléotidique selon l'une des revendications précédentes, caractérisée en ce qu'elle est liée à un groupe prosthétique choisi parmi des polyaminoacides, des lipides, des hormones ou des peptides, pour améliorer l'introduction dans la cellule et/ou la localisation cellulaire spécifique de la structure oligonucléotidique.

19. Procédé de coupure d'une séquence d'acides nucléiques cible à l'aide d'une structure oligonucléotidique catalytique synthétique selon l'une des revendications 1 à 18.

20. Procédé selon la revendication 19, caractérisé en ce que la séquence d'acides nucléiques cible est une molécule différente de la structure oligonucléotidique catalytique synthétique.

21. Procédé selon l'une des revendications 19 ou 20, caractérisé en ce que l'on coupe une séquence d'acides nucléiques cible de formule générale (IV) l'aide d'un oligonucléotide de formule générale (II), avec formation d'un intermédiaire ayant la structure suivante: 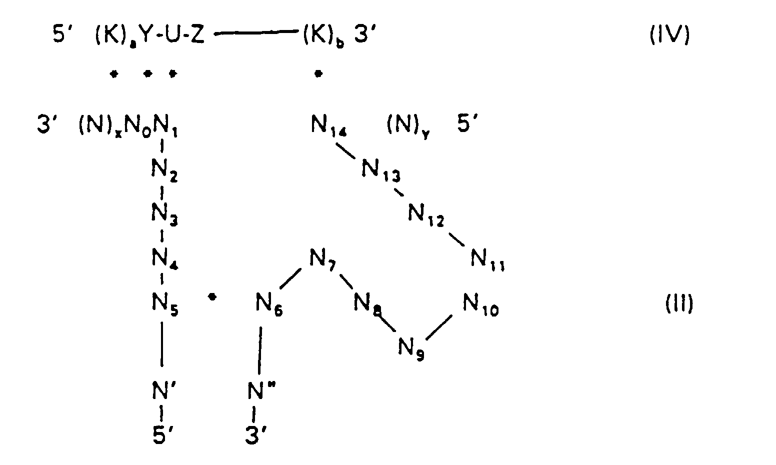 dans laquelle
les symboles N, N', N", x, y et * ont la définition donnée dans la revendication 1,
et K, Y, U et Z représentent des nucléotides de la séquence d'acides nucléiques cible, dans laquelle
U est l'uridine,
Z est un ribonucléotide non modifié choisi dans le groupe constitué par l'adénosine, la cytidine et l'uridine,
K et Y sont des nucléotides quelconques,
a est ≥ 1 et b ≥ 3,
et la coupure de la séquence d'acides nucléiques cible s'effectue du côté 3' de la séquence de nucléotides YUZ, et il existe éventuellement une liaison chimique entre l'extrémité 5' de la séquence d'acides nucléiques cible (IV) et l'extrémité 3' de l'oligonucléotide (II) au niveau de (N)ₓ ou entre l'extrémité 3' de la séquence d'acides nucléiques cible (IV) et l'extrémité 5' de l'oligonucléotide (II) au niveau de (N)_{y}.

22. Procédé selon la revendication 21, caractérisé en ce que Y est la guanosine.

23. Procédé selon la revendication 21 ou 22, caractérisé en ce que Z est l'adénosine ou la cytidine.

24. Procédé selon l'une des revendications 19 à 23, caractérisé en ce que la séquence d'acides nucléiques cible est un acide ribonucléique.

25. Procédé selon l'une des revendications 19 à 24, caractérisé en ce que l'on effectue la coupure en présence d'un cation divalent, en particulier de Mg²⁺.

26. Procédé selon l'une des revendications 19 à 24, caractérisé en ce que l'on effectue la coupure à un pH d'environ 7 à 9.

27. Utilisation d'une structure oligonucléotidique selon l'une des revendications 1 à 18 pour la coupure catalytique d'une séquence d'acides nucléiques cible.

28. Médicament, caractérisé en ce qu'il contient comme substance active une structure oligonucléotidique selon l'une des revendications 1 à 18, éventuellement avec des supports, des agents auxiliaires, des charges et/ou des diluants pharmaceutiques classiques.

29. Procédé de préparation d'un médicament pour une thérapie antivirale chez l'homme, les animaux ou les végétaux, caractérisé en ce que l'on utilise comme substance active une structure oligonucléotidique selon l'une des revendications 1 à 18.

30. Réactif de diagnostic, caractérisé en ce qu'il contient comme constituant une structure oligonucléotidique selon l'une des revendications 1 à 18.

31. Procédé de préparation d'un réactif de diagnostic pour une procédure d'analyse génétique, caractérisé en ce qu'il contient comme constituant une structure oligonucléotidique selon l'une des revendications 1 à 18.

## Revendications (Revendications pour l'(les) Etat(s) contractant(s) suivant(s): ES)

1. Procédé de préparation de structures oligonucléotidiques catalytiques synthétiques à partir d'unités monomères, dans lequel la structure oligonucléotidique est appropriée à l'hybridation avec une séquence d'acides nucléiques cible et à sa coupure, et a une structure oligonucléotidique ayant la formule structurale générale (II): 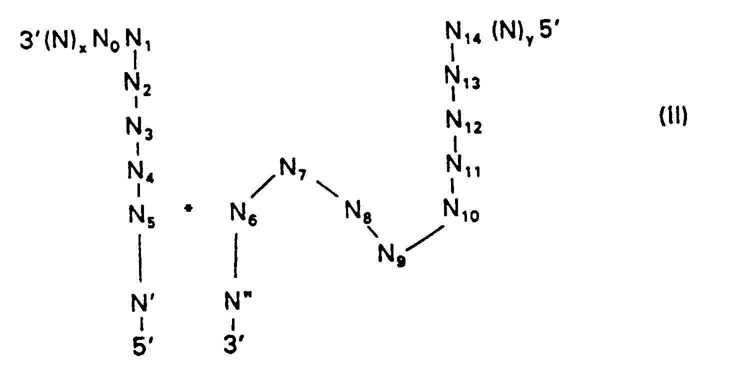 dans laquelle
les N représentent chacun un nucléotide de formule structurale générale (I),
x et y peuvent être identiques ou différents et x est ≥ 1 et y ≥ 2,
N₅ et N₆ sont des nucléotides complémentaires l'un de l'autre et * représente un appartement de bases,
N' et N" représentent deux séquences de nucléotides contenant des nucléotides au moins partiellement complémentaires les uns des autres, de façon à permettre un appariement de bases stable entre les deux séquences de nucléotides,
ou bien
N' et N" représentent ensemble une seule séquence de nucléotides, au moins une partie de la séquence pouvant former un tronc double brin par appartement de bases entre des nucléotides complémentaires,
et un ou plusieurs nucléotides N supplémentaires peuvent éventuellement être insérés après N₇ et/ou N₉,
et la structure oligonucléotidique contient des nucléotides ayant la formule structurale générale (I): 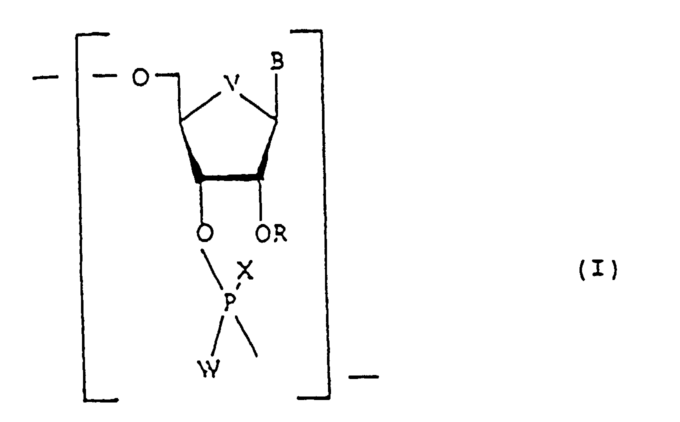
dans laquelle
B représente une base de nucléoside choisie en particulier dans le groupe constitué par les restes adénine-9-yle (A), cytosine-1-yle (C), guanine-9-yle (G), uracile-1-yle (U), uracile-5-yle (ψ), hypoxanthine-9-yle (I), thymine-1-yle (T) et 2-aminoadénine-9-yle,
V est indépendamment, dans chaque nucléotide, un atome d'oxygène ou un groupe CH₂,
X et W peuvent être identiques ou différents dans chaque nucléotide, et représentent indépendamment l'un de l'autre un atome d'oxygène ou de soufre ou un groupe NH₂, alkyle ou alcoxy de 1 à 10, de préférence 1 à 4 atomes de carbone,
R est un atome d'hydrogène ou un groupe alkyle, alcényle ou alcynyle de 1 à 10 atomes de carbone linéaire ou ramifié, éventuellement substitué par des restes halogéno, cyano, isocyano, nitro, amino, carboxyle, hydroxyle et/ou mercapto,
et, dans les nucléotides N₁, N₄, N₈ et N₁₁ de la formule (II), le reste R de la formule (I) est un atome d'hydrogène, et, dans au moins l'un des nucléotides N₂, N₃, N₅, N₆, N₇, N₉, N₁₀, N₁₂, N₁₁ et N₁₄ de la formule (II), le reste R de la formule (I) est différent d'un atome d'hydrogène.

2. Procédé selon la revendication 1, caractérisé en ce que, dans les nucléotides N₁, N₄, N₈ et N₁₁, les restes B de la formule (I) sont respectivement A ou 2-aminoadénine-9-yle, G, G et G.

3. Procédé selon l'une des revendications précédentes, caractérisé en ce que les restes V, W et X de la formule (I) sont des atomes d'oxygène.

4. Procédé selon l'une des revendications précédentes, caractérisé en ce que, dans la formule (I), les restes R différents de l'hydrogène contiennent de 1 à 6 atomes de carbone.

5. Procédé selon la revendication 4, caractérisé en ce que, dans la formule (I), les restes R différents de l'hydrogène sont choisis dans le groupe constitué par les restes méthyle, éthyle, propyle, allyle, diméthylallyle, butyle ou cyanométhyle.

6. Procédé selon l'une des revendications précédentes, caractérisé en ce que la structure oligonucléotidique a la formule structurale générale (III): 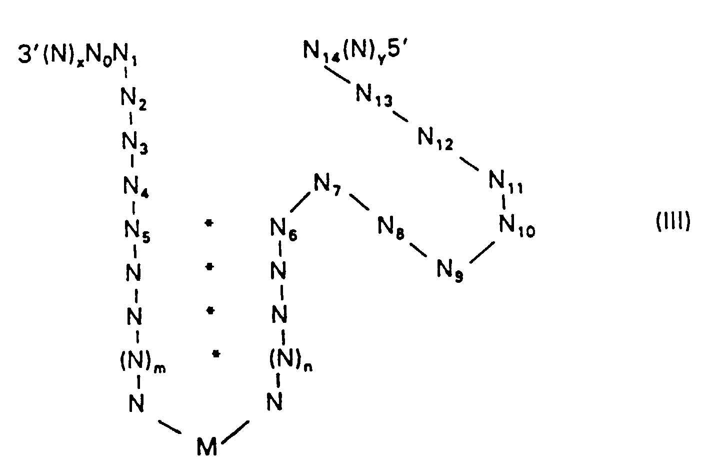 dans laquelle
N, x et y ont la définition donnée dans la revendication 1,
M représente une liaison chimique ou une séquence de nucléotides (N)ₐ dans laquelle a est ≥ 1, m et n sont identiques ou différents et un ou plusieurs nucléotides supplémentaires peuvent éventuellement être insérés après N₇ et/ou N₉.

7. Procédé selon la revendication 6, caractérisé en ce que, dans la formule (III), les restes R représentent l'hydrogène dans les nucléotides N₁, N₄, N₈, N₁₀, N₁₁ et N₁₂.

8. Procédé selon la revendication 6 ou 7, caractérisé en ce que, dans la formule (III), les restes R sont différents de l'hydrogène dans tous les nucléotides à l'exception de N₁, N₄, N₈, N₁₀, N₁₁ et N₁₂.

9. Procédé selon l'une des revendications précédentes, caractérisé en ce que, dans la formule (I), les restes V, W et X sont des atomes d'oxygène et en ce que, dans les nucléotides N₁ à N₁₄ de la formule (I) ou (III), les restes B et R ont les significations suivantes:
N₁: B = A et R = H,
N₂ : B = A et R = allyle,
N₃ : B = A et R = allyle,
N₄ : B = G et R = H,
N₅ : B = C et R = allyle,
N₆ : B = G et R = allyle,
N₇ : B = A et R = allyle,
N₈ : B = G et R = H,
N₉ : B = U et R = allyle,
N₁₀ : B = A et R = H,
N₁₁ : B = G et R = H,
N₁₂ : B = U et R = H,
N₁₃ : B = C et R = allyle,
N₁₄ : B = U et R = allyle.

10. Procédé selon l'une des revendications 1 à 8, caractérisé en ce que, dans la formule (I), les restes V, W et X sont des atomes d'oxygène, avec l'exception selon laquelle la liaison entre N₁₁ et N₁₂ est un groupe phosphorothioate (X = O et W = S), et en ce que, dans les nucléotides N₁ à N₁₄ de la formule (I) ou (III), les restes B et R ont la signification donnée dans la revendication 9.

11. Procédé selon l'une des revendications 1 à 8, caractérisé en ce que, dans la formule (I), les restes V, W et X sont des atomes d'oxygène, et en ce que, dans les nucléotides N₁ à N₁₄ de la formule (I) ou (III), les restes B ont la signification donnée dans la revendication 9, et en ce que, pour N₁, N₄, N₈, N₁₀, N₁₁ et N₁₂, R = H; pour N₉, R = 3,3-diméthylallyle et, pour N₂, N₃, N₅, N₆, N₇, N₁₃ et N₁₄, R = allyle.

12. Procédé selon l'une des revendications 1 à 8, caractérisé en ce que, dans la formule (I), les restes V, W et X sont des atomes d'oxygène, et en ce que, dans les nucléotides N₁ à N₁₄ de la formule (I) ou (III), les restes B ont la signification donnée dans la revendication 9, et en ce que, pour N₁, N₄, N₈, N₁₀, N₁₁ et N₁₂, R = H; pour N₃, R = 3,3-diméthylallyle et, pour N₂, N₅, N₆, N₇,N₉, N₁₃ et N₁₄, R = allyle.

13. Procédé selon l'une des revendications 1 à 8, caractérisé en ce que, dans la formule (I), les restes V, W et X sont des atomes d'oxygène, et en ce que, dans les nucléotides N₁ à N₁₄ de la formule (I) ou (III), les restes B ont la signification donnée dans la revendication 9, et en ce que, pour N₁, N₄, N₈, N₁₀, N₁₁ et N₁₂, R = H; pour N₂, N₃, N₇, N₉ et N₁₃, R = cyanométhyle et, pour N₅, N₆ et N₁₄, R = allyle.

14. Procédé selon l'une des revendications 1 à 8, caractérisé en ce que, dans la formule (I), les restes V, W et X sont des atomes d'oxygène, et en ce que, dans les nucléotides N₁ à N₁₄ de la formule (I) ou (III), les restes B ont la signification donnée dans la revendication 9, et en ce que, pour N₁, N₄, N₈, N₁₀, N₁₁ et N₁₂, R = H; pour N₅ et N₆, R = butyle et pour N₂, N₃, N₇, N₉, N₁₃ et N₁₄, R = allyle; R étant éventuellement butyle pour un ou plusieurs des restes N qui se trouvent sous une forme à bases appariées dans la formule (III).

15. Procédé selon l'une des revendications précédentes, caractérisé en ce que des 3'-désoxyribonucléotides et/ou des 3'-O-alkylribonucléctides se trouvent aux extrémités 3' libres de la structure oligonucléotidique.

16. Procédé selon l'une des revendications précédentes, caractérisé en ce que la structure oligonucléotidique contient en outre, pour la stabilisation de sa configuration dans l'espace, des nucléotides dont les bases de nucléoside sont modifiées par un agent de réticulation transversale.

17. Procédé selon la revendication 16, caractérisé en ce que l'agent de réticulation transversale est le psoralène ou un dérivé de psoralène.

18. Procédé selon l'une des revendications précédentes, caractérisé en ce que la structure oligonucléotidique est liée à un groupe prosthétique choisi parmi des polyaminoacides, des lipides, des hormones ou des peptides, pour améliorer son introduction dans la cellule et/ou sa localisation cellulaire spécifique.

19. Procédé de coupure d'une séquence d'acides nucléiques cible à l'aide d'une structure oligonucléotidique catalytique synthétique préparée par le procédé selon l'une des revendications 1 à 18.

20. Procédé selon la revendication 19, caractérisé en ce que la séquence d'acides nucléiques cible est une molécule différente de la structure oligonucléotidique catalytique synthétique.

21. Procédé selon l'une des revendications 19 ou 20, caractérisé en ce que l'on coupe une séquence d'acides nucléiques cible de formule générale (IV) à l'aide d'un oligonucléotide de formule générale (II), avec formation d'un intermédiaire ayant la structure suivante: 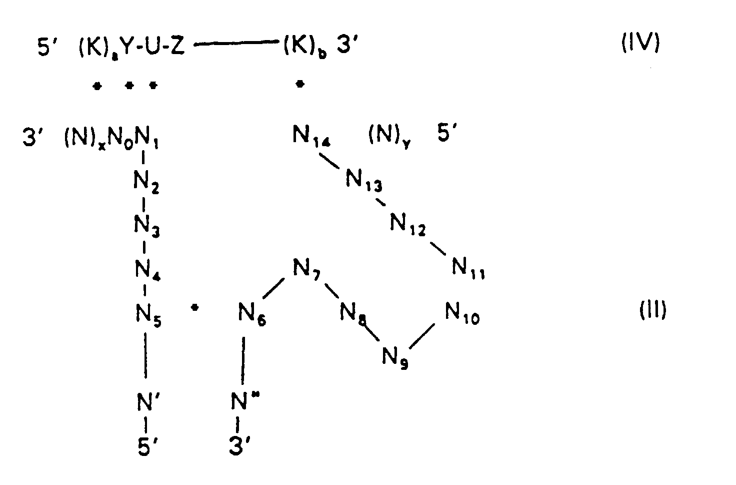 dans laquelle
les symboles N, N', N", x, y et * ont la définition donnée dans la revendication 1,
et K, Y, U et Z représentent des nucléotides de la séquence d'acides nucléiques cible, dans laquelle
U est l'uridine,
Z est un ribonucléotide non modifié choisi dans le groupe constitué par l'adénosine, la cytidine et l'uridine,
K et Y sont des nucléotides quelconques,
a est ≥ 1 et b ≥ 3,
et la coupure de la séquence d'acides nucléiques cible s'effectue du côté 3' de la séquence de nucléotides YUZ, et il existe éventuellement une liaison chimique entre l'extrémité 5' de la séquence d'acides nucléiques cible (IV) et l'extrémité 3' de l'oligonucléotide (II) au niveau de (N)ₓ ou entre l'extrémité 3' de la séquence d'acides nucléiques cible (IV) et l'extrémité 5' de l'oligonucléotide (II) au niveau de (N)_{y}.

22. Procédé selon la revendication 21, caractérisé en ce que Y est la guanosine.

23. Procédé selon la revendication 21 ou 22, caractérisé en ce que Z est l'adénosine ou la cytidine.

24. Procédé selon l'une des revendications 19 à 23, caractérisé en ce que la séquence d'acides nucléiques cible est un acide ribonucléique.

25. Procédé selon l'une des revendications 19 à 24, caractérisé en ce que l'on effectue la coupure en présence d'un cation divalent, en particulier de Mg²⁺.

26. Procédé selon l'une des revendications 19 à 24, caractérisé en ce que l'on effectue la coupure à un pH d'environ 7 à 9.

27. Utilisation d'une structure oligonucléotidique préparée par un procédé selon l'une des revendications 1 à 18 pour la coupure catalytique d'une séquence d'acides nucléiques cible.

28. Procédé de préparation d'un médicament caractérisé en ce que l'on utilise comme substance active une structure oligonucléotidique préparée par un procédé selon l'une des revendications 1 à 18, et on la formule éventuellement avec des supports, des agents auxiliaires, des charges et/ou des diluants pharmaceutiques classiques.

29. Procédé de préparation d'un médicament pour une thérapie antivirale chez l'homme, les animaux ou les végétaux, caractérisé en ce que l'on utilise comme substance active une structure oligonucléotidique préparée par un procédé selon l'une des revendications 1 à 18.

30. Réactif de diagnostic, caractérisé en ce qu'il contient comme constituant une structure oligonucléotidique préparée par un procédé selon l'une des revendications 1 à 18.

31. Procédé de préparation d'un réactif de diagnostic pour une procédure d'analyse génétique, caractérisé en ce qu'il contient comme constituant une structure oligonucléotidique préparée par un procédé selon l'une des revendications 1 à 18.
